# EUROPEAN PATENT APPLICATION

(11) **EP 4 026 533 A1**
(43) Date of publication of application: **13.07.2022**
(21) Application number: 21150458.4
(22) Date of filing: 07.01.2021
(51) Int. Cl.: A61K 8/31, A61Q 19/00

(54) **C13 - HYDROCARBON MIXTURE AND USE THEREOF**

(71) Applicant: BASF SE, 67056 Ludwigshafen (DE)
(72) Inventor: Dierker, Markus, 40589 Duesseldorf (DE); Zhu, Ben Chuan, 200137 Shanghai (CN); Masaki, Koichi, 306-0213 Koga-Shi (JP); Wakebe, Takanori, 306-0213 Koga-Shi (JP); Ernst, Martin, 67056 Ludwigshafen (DE)
(74) Representative: BASF IP Association

(57) **Abstract**

The present invention relates to a specific hydrocarbon mixture comprising
at least 95% by weight of saturated linear C-13 hydrocarbon based on the sum of the hydrocarbons in the hydrocarbon mixture,
characterized in that the amount of C11/C12 is 0.05 to 1.5 % by weight based on the sum of the hydrocarbons in the hydrocarbon mixture and
the amount of C14 to C17 is 0.2 to 3.5 % by weight based on the sum of the hydrocarbons in the hydrocarbon mixture and the use thereof in cosmetic and/or pharmaceutical formulations, and to cosmetic formulations comprising the hydrocarbon mixture.

## Description

The present invention relates to a specific hydrocarbon mixture, to the use thereof in cosmetic and/or pharmaceutical formulations, and to cosmetic formulations comprising the hydrocarbon mixture.

### State of the Art

Volatile linear hydrocarbons have successfully been used in cosmetic compositions as so called "light emollients".

Obtained on the basis of renewable raw materials they are ecologically and toxicologically superior to hydrocarbons from petrochemical processes or silicones and fulfill the growing needs of environmental-friendly cosmetic ingredients.

The PCT-application WO 2007/068371 relates to a method for producing these linear saturated alkanes from primary alcohols the C chain of which has one carbon atom more than the alkane, by reductive dehydroxymethylation of the primary alcohols in the presence of hydrogen and a catalyst. The primary alcohols used are selected from fatty alcohols with 8 to 24 carbon atoms. High-purity hydrocarbons with a particular chain length can be produced from the reaction mixtures obtained thereby, preferably after purification of the crude products, for example, after fractional distillation, and-again preferably-deodorization.

These hydrocarbons with a particular chain length thus obtained have either been used as individual components in cosmetic formulations as so-called light emollients or may be mixed in a particular manner in order to be able to establish special properties such as, for example, spreading behavior, volatility or even a flash point. Specifically emollient mixtures of the C11- and C13- chain length as disclosed in the International patent application WO 2008/155057 have shown a favorable profile to substitute silicone oils. They exhibit improved sensory properties and skin tolerability compared to the hydrocarbon mixtures of the prior art.

From European application no. EP 2324816 A1 it is known to combine linear hydrocarbons of C11- and C13-chain length with fragrances. As cited therein each fragrance is the combination of various odorous substances which evaporate at different periods. A fragrance has a so called "top note" which is the first odor diffusing upon application of the perfume or when opening the container the container, a "heart note or body" which corresponds to the complete fragrance (emission for several hours after the "top note") and a "base note" which is the most persistent odor (emission for several hours after the "heart note"). The persistence of the base note corresponds to the persistence of the perfume. Unfortunately this base note, body and top note are changed by using C11- and C13-hydrocarbon mixtures since this hydrocarbon distribution has its own scent and specific volatility.

Therefore, it is of particular interest to provide skin tolerable raw materials with no odor, so that these hydrocarbons could especially be used in fragrances, perfumes, decorative cosmetics and formulations for sensitive skin. It was desirable, more particularly, to provide raw materials which are suitable as solvents for perfume oils and fragrances.

On the other hand odorless and perfume-free compositions do not need perfumes and fragrances to cover an unwanted odor, so that an odorless raw material is needed which could preferably be used for perfume-free formulations and cosmetics for sensitive skin.
It was a further object to provide raw materials which enable a stable formulation with active ingredients for decorative cosmetics as there also exists a need for the volatile solvents for formulating decorative cosmetic compositions rich in pigments and dyes.

Of particular interest is the provision of novel raw materials which enable a sensorily advantageous impression in cosmetics formulations and are well tolerable for sensitive skin. Owing to the site of application which is mainly the face, increased demands are made on the sensory properties, especially the volatility, on formulations in decorative cosmetics, for example lipsticks, eyeshadow, mascara, nail varnish, etc., in order that these products do not give the impression of "heaviness". In addition, good dispersibility of pigments is desirable in these products.

### Description of the Invention

The aforementioned objects are solved with a hydrocarbon mixture comprising at least 95 % by weight of a linear C13 hydrocarbon based on the sum of hydrocarbons, characterized in that the amount of C11/C12 is 0.05 to 1.5 % by weight based on the sum of the hydrocarbons in the hydrocarbon mixture and the amount of C14 to C17 is 0.2 to 3.5 % by weight based on the sum of the hydrocarbons in the hydrocarbon mixture.

This specific hydrocarbon distribution has shown advantageous properties in view of its odor and could preferably be used in cosmetic compositions in combination with perfumes, perfume oils and fragrances without changing the profile of the perfume containing composition.

In addition it could specifically be used in perfume-free compositions because masking the odor of the emollient is not necessary. For compositions without any fragrances or perfumes odorless raw material is needed, so that the hydrocarbon mixture of the current invention could preferably be used for perfume-free formulations and thus for cosmetics for sensitive skin.

The pure C13-hydrocarbon had the same odor profile but requires many purification steps and a time and energy consuming manufacturing process. In addition the sensory properties and spreadability of the hydrocarbon mixture of the invention, which are important for the use in cosmetic and personal care compositions, are advantageous due to its light non-greasy, non-waxy impression after dermal application.

Preferably the hydrocarbon mixture comprises 95 to 99.5 % by weight of saturated linear C-13 hydrocarbon based on the sum of the hydrocarbons and the amount of C11/C12 is 0.1 to 1.0 % by weight based on the sum of the hydrocarbons in the hydrocarbon mixture and the amount of C14 to C17 is 0.3 to 3.0 % by weight based on the sum of the hydrocarbons in the hydrocarbon mixture.

In another embodiment of the invention the hydrocarbon mixture comprises at least 97 % by weight of saturated linear C-13 hydrocarbon based on the sum of the hydrocarbons and the amount of C11/C12 is 0.1 to 0.5 % by weight based on the sum of the hydrocarbons in the hydrocarbon mixture and the amount of C14 to C17 is 0.4 to 2.5 % by weight based on the sum of the hydrocarbons in the hydrocarbon mixture.

The parameter "sum of the hydrocarbons" includes all hydrocarbons present in the mixture, irrespective of their carbon number.

Hydrocarbons refer to organic compounds which consist only of carbon and hydrogen. They include both cyclic and acyclic (= aliphatic) compounds. They include both saturated and mono- or polyunsaturated compounds. The hydrocarbons may be linear or branched. According to the number of carbon atoms in the hydrocarbon, the hydrocarbons can be divided into odd-numbered hydrocarbons (for example nonane, undecane, tridecane) or even-numbered hydrocarbons (for example octane, dodecane, tetradecane). According to the type of branching, the hydrocarbons can be divided into linear (= unbranched) or branched hydrocarbons. Saturated aliphatic hydrocarbons are also referred to as paraffins.

A "hydrocarbon mixture" in the context of the invention is understood to mean mixtures of hydrocarbons which contain up to 5 % by weight of substances which are not hydrocarbons. The percentages by weight of the linear C13 hydrocarbons are based in each case on the sum of the hydrocarbons present in the mixture. The nonhydrocarbons which are present up to 5% by weight are not considered for this calculation.

The substances which are not hydrocarbons and which may be present up to 5 % by weight, preferably up to 3 % by weight, more preferably up to 1% by weight and specifically up to 0.1 % by weight based on the weight of the hydrocarbon mixture are, for example, fatty alcohols, which remain in the hydrocarbon mixture as unconverted reactants.

The hydrocarbon mixture according to the invention is characterized in that the sum of the hydrocarbons having a **carbon chain length greater than or equal to 17** is less than or equal to 0.5 % by weight, based on the sum of the hydrocarbons.

The hydrocarbon mixture according to the invention is characterized in that the sum of the hydrocarbons having a **carbon chain length less than or equal to 10 is less than** or equal to 0.5 % by weight, based on the sum of the hydrocarbons.

In a preferred embodiment of the invention, the inventive hydrocarbon mixture comprises less than or equal to 3 % by weight, preferably less than or equal to 2 % by weight, more preferably less than or equal to 1 % by weight and especially less than or equal to 0.5 % by weight, of **branched hydrocarbons,** based on the sum of the hydrocarbons.

In a preferred embodiment of the invention, the inventive hydrocarbon mixture comprises less than 1%, especially less than 0.1% and especially less than 0.01% by weight of **aromatic hydrocarbons,** based on the sum of the hydrocarbons.

In a preferred embodiment of the invention, the inventive hydrocarbon mixture comprises less than or equal to 1%, especially less than or equal to 0.1% and especially less than or equal to 0.03% by weight of **unsaturated hydrocarbons,** based on the sum of the hydrocarbons. In a preferred embodiment, the inventive hydrocarbon mixtures comprise less than or equal to 1.5 % by weight, preferably less than or equal to 1 % by weight, more preferably less than or equal to 0.5 % by weight of C-12 hydrocarbons, based on the sum of the hydrocarbons.

The hydrocarbon mixture according to any of the preceding claims, characterized in that the amount of tetradecanol is less than or equal to 1 % by weight, preferably less than or equal to 0.5 % by weight, more preferably less than or equal to 0.1 % by weight of based on the weight of the hydrocarbon mixture.

A preferred embodiment of the invention relates to hydrocarbon mixtures, wherein the mixture comprises
at least 95 % by weight of C13-hydrocarbons, preferably linear C13-hydrocarbons,
0.01 - 1.5 % by weight of undecane,
0.01 - 1.5 % by weight of dodecane
0.1 - 2.0 % by weight of tetradecane and
0.01 - 1.5 % by weight of pentadecane
based on the sum of the hydrocarbons in the hydrocarbon mixture, preferably
at least 95 % by weight of C13-hydrocarbons, preferably linear C13-hydrocarbons,
0.01 - 1.0 % by weight of undecane,
0.01 - 1.0 % by weight of dodecane
0.1 - 2.0 % by weight of tetradecane and
0.1 - 1.5 % by weight of pentadecane
based on the sum of the hydrocarbons in the hydrocarbon mixture, more preferably
at least 95 % by weight of C13-hydrocarbons, preferably linear C13-hydrocarbons,
0.01 - 0.7 % by weight of undecane,
0.01 - 0.7 % by weight of dodecane
0.1 - 2.0 % by weight of tetradecane and
0.1 - 1.5 % by weight of pentadecane
based on the sum of the hydrocarbons in the hydrocarbon mixture.

Particular preference is given to hydrocarbon mixtures which comprise
at least 95 % by weight of linear C13-hydrocarbons,
0.01 - 0.5 % by weight of undecane,
0.01 - 0.5 % by weight of dodecane
0.1 - 1.5 % by weight of tetradecane and
0.1 - 1.2 % by weight of pentadecane
based on the sum of the hydrocarbons in the hydrocarbon mixture.

The inventive hydrocarbon mixtures are suitable especially for use in cosmetic, personal care and/or pharmaceutical formulations, especially as oil bodies, toner, cleanser, conditioner, solvent, dispersant and/or emollient.

The invention further provides a process for producing a cosmetic and/or pharmaceutical formulation, wherein the hydrocarbon mixture according to the invention is added to a cosmetically and/or pharmaceutically suitable carrier.

### Production of the hydrocarbon mixtures

The fatty alcohols with the specific chain length needed may be produced in known manner from renewable raw materials, such as coconut oil, palm oil or palm kernel oil for example, by transesterification with methanol and subsequent hydrogenation. Besides pure fatty alcohols, other linear or branched, monohydric or polyhydric alcohols, alcohol mixtures or derivatized alcohols produced on an industrial scale may also be used in principle. In a preferred embodiment, the primary alcohols used correspond to the general formula R-OH, where R is a saturated linear alkyl group containing 14 carbon atoms.

The inventive hydrocarbon mixture is preferably obtained by reductive demethylation by methods known to those skilled in the art. A particularly suitable process for producing the inventive hydrocarbon mixtures is the process for reductive dehydroxymethylation proceeding from fatty alcohols of vegetable origin, described in international application WO 2007/068371.

In this process, for example, specifically selected purified C14 fatty alcohols can be subjected individually to the process described, and the C13 hydrocarbons thus obtained can be purified by further distillation. It is preferred, however, to subject a mixture of C14 fatty alcohols which comprises at least 95 % by weight of C14 fatty alcohols to the reductive dehydroxymethylation, such that the reaction product obtained directly is the inventive hydrocarbon mixture.

This procedure avoids complex fractionation procedures and the need of high separation efficiency but still achieving a product with sufficient purity by conducting a simple distillation without energy and time consuming separation steps. The product can directly be used in cosmetic and/or pharmaceutical formulations without complex purification steps.

### Cosmetic and/or pharmaceutical formulations

The inventive hydrocarbon mixtures comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, are suitable for use in cosmetic formulations for care of skin and/or hair, especially for use in cosmetic formulations for sun protection.

Due to their odorless properties the inventive hydrocarbon mixtures are especially suitable for formulations comprising perfumes, perfume oils or other fragrances and are primarily incorporated into compositions of decorative cosmetic formulations, for example lipsticks, lip gloss, foundations, coversticks, pressed and loose powders, eyeshadow, mascara, eye pencils, nail varnish, and in makeup formulations of any kind.

The hydrocarbon mixtures of the invention are suitable for use in formulations for cleaning skin after use of decorative cosmetics such as make-up remover and for cleaning skin and/or hair, for example shampoos, shower gels, bath additives, especially for conditioners as well as compositions such as eaux fraîches, eaux de parfum, eaux de toilette, elixirs or fragrance extracts and aftershave lotions.

The hydrocarbon mixtures of the invention are also suitable for producing finely divided emulsions, for example nanoemulsions, microemulsions or PIT emulsions. In such finely divided emulsions, the oil droplets are generally present with a diameter in the range from 10 to 1000 nm, preferably 100 to 500 nm.

Another subject of the invention are cosmetic and/or pharmaceutical formulations comprising 0.1 to 80% by weight of a hydrocarbon mixture based on the cosmetic and/or pharmaceutical formulation, which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture.

A preferred embodiment of the invention relates to cosmetic and/or pharmaceutical formulations comprising 0.1 to 80% by weight, preferably 0.5 to 50% by weight, more preferably 5 to 25 % and most preferably 1 to 5 % by weight of the hydrocarbon mixture comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture.

The parameter "sum of the hydrocarbons" encompasses all hydrocarbons present in the cosmetic and/or pharmaceutical formulation, irrespective of their carbon number.
The term "% by weight of hydrocarbons" or "% by weight of the hydrocarbon mixture" always relates - unless stated otherwise - to the total weight of the cosmetic and/or pharmaceutical formulation.

These cosmetic and/or pharmaceutical formulations may accordingly comprise further hydrocarbons, for example paraffins or waxes, provided that the sum of the linear C13 hydrocarbons is greater than or equal to 95 % by weight, based on the sum of the hydrocarbons.

The cosmetic compositions according to the invention can be in particular formulations for bodycare, facecare, suncare and haircare as well as decorative cosmetics e.g. a body milk, creams, lotions, aftershave lotions, sprayable emulsions, tonics and scented waters, products for eliminating body odor such as deodorants and antiperspirants, make-up removers, conditioners, styling products. The solubilizer composition can also be used in formulations containing further surfactants such as e.g. foam and shower baths, hair shampoos and care rinses.

An especially preferred embodiment of the invention relates to cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture comprising 95 to 99.5 % by weight of saturated linear C-13 hydrocarbon based on the sum of the hydrocarbons, saturated linear C11/C12 hydrocarbons in an amount of 0.1 to 1.0 % by weight based on the sum of the hydrocarbons in the hydrocarbon mixture and saturated linear C14 to C17-hydrocarbons in an amount of 0.3 to 3.0 % by weight based on the sum of the hydrocarbons in the hydrocarbon mixture.

Another especially preferred embodiment of the invention relates to cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture comprising 95 to 99.5 % by weight of saturated linear C-13 hydrocarbon based on the sum of the hydrocarbons, saturated linear C11/C12 hydrocarbons in an amount of 0.1 to 1.0 % by weight based on the sum of the hydrocarbons in the hydrocarbon mixture and saturated linear C14 to C17-hydrocarbons in an amount of 0.3 to 3.0 % by weight based on the sum of the hydrocarbons in the hydrocarbon mixture and which is free of fragrances, perfumes or perfume oils.

In a preferred embodiment, the cosmetic and/or pharmaceutical formulations comprise 0.1 to 80% by weight, preferably 0.5 to 50% by weight, more preferably 5 to 25 % and most preferably 1 to 5 % by weight of a hydrocarbon mixture, comprising at least 95 % by weight of a linear C13 hydrocarbon based on the sum of hydrocarbons, C11/C12 - hydrocarbons in an amount of 0.05 to 1.5 % by weight based on the sum of the hydrocarbons in the hydrocarbon mixture and C14 to C17 - hydrocarbons in an amount of 0.2 to 3.5 % by weight based on the sum of the hydrocarbons in the hydrocarbon mixture, characterized in that the amount of tetradecanol is less than or equal to 1 % by weight based on the hydrocarbon mixture.

Particular preference is given to cosmetic and/or pharmaceutical formulations containing 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture and which comprises less than 3 % by weight, preferably less than or equal to 2 % by weight, preferably less than or equal to 1 % by weight, especially less than or equal to 0.5 % by weight, of **branched hydrocarbons** based on the sum of the hydrocarbons.

Particular preference is given to cosmetic and/or pharmaceutical formulations containing 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture and which comprises less than 1 % by weight, preferably less than or equal to 0.1 % by weight of **aromatic hydrocarbons** based on the sum of the hydrocarbons.

Particular preference is given to cosmetic and/or pharmaceutical formulations containing 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture and which comprises less than or equal to 1%, especially less than or equal to 0.1% and especially less than or equal to 0.01% by weight of **unsaturated hydrocarbons**, based on the sum of the hydrocarbons in the hydrocarbon mixture.

Preferably these cosmetic and/or pharmaceutical formulations are comprising additional auxiliaries and additives selected from the group consisting of surface-active substances (surfactants, emulsifiers), other oil components, pearlizing waxes, consistency factors, thickeners, superfatting agents, stabilizers, polymers, fats, waxes, lecithins, phospholipids, biogenic agents, UV protection factors, antioxidants, deodorizers, antiperspirants, antidandruff agents, film formers, swelling agents, insect repellents, self-tanning agents, tyrosinase inhibitors (depigmenting agents), hydrotropes, solubilizers, preservatives, perfume oils, pigments, dyes and mixtures thereof.

More preferably the cosmetic formulations contain 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture and at least one perfume, perfume oil or fragrance as ingredient and /or at least one UV light protection filter and/or pigments or dyes.

In another embodiment of the invention the cosmetic formulations are free of perfumes, fragrances and perfume oils and contain 0.1 to 80% by weight, preferably 0.5 to 50% by weight, more preferably 1 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture and additional additives selected from the group consisting of surface-active substances (surfactants, emulsifiers), other oil components, pearlizing waxes, consistency factors, thickeners, superfatting agents, stabilizers, polymers, fats, waxes, lecithins, phospholipids, biogenic agents, UV protection factors, antioxidants, deodorizers, antiperspirants, antidandruff agents, film formers, swelling agents, insect repellents, self-tanning agents, tyrosinase inhibitors (depigmenting agents), hydrotropes, solubilizers, preservatives, pigments, dyes and mixtures thereof.

### Perfume, perfume oils or fragrances

Perfume oils include mixtures of natural and synthetic origin. Natural odorants are extracts of flowers, stems and leaves, fruit, fruit shells, roots, wood, herbs and grasses, needles and branches, resins and balsams or as well animal raw materials, such as, for example, civet and castoreum, and also synthetic odorant compounds of the ester, ether, aldehyde, ketone, alcohol and hydrocarbon type.

Perfume oils comprise extracts of flowers such as roses, lilies, lavender, calendula, chamomile, linden blossom, lilly of the valley, jasmine, neroli, passion flower, ylang-ylang; of stems and flowers such as geranium, patchouli, petitgrain; of fruits such as anise, cloves, coriander, caraway, juniper, mango, peach, vanilla; of fruit peels such as bergamot, lemons, oranges; of roots such as mace, angelica, celery, cardamom, costus, iris, calmus; of woods such as pine, sandal, gujak, cedar, rosewood; of herbs and grasses such as tarragon, lemongrass, sage, thyme, rosemary, mint, lemon balm, cinnamon leaves; of needles and twigs such as spruce, fir, pine, mountain pines or of resins and balms such as galbanum, elemi, benzoin, myrrh, olibanum, opoponax.

Fragrances can also include synthetic products of an ester, ether, aldehyde, ketone, alcohols or hydrocarbon.

Fragrance compounds of the ester type are for example benzyl acetate, phenoxyethyl isobutyrate, p-tert.-butylcyclohexyl acetate, linalyl acetate, dimethylbenzylcarbinylacetate, phenylethyl acetate, linalyl benzoate, benzyl formate, ethyl methylphenyl glycinate, allyl cyclohexyl propionate, styrallyl propionate and benzyl salicylate.
Ethers include, for example, benzyl ethyl ether, (dihydro) rose oxide.

Aldehydes can be selected from linear alkanals with 8 to 18 C-atoms, citral, citronellal, citronellyloxyacetaldehyde, cyclamenaldehyde, hydroxycitronellal, lilial and bourgeonal and ketones are ionones, alpha-isomethylionon and methylcedrylketon.
Alcohols include anethole, citronellol, eugenol, geraniol, linalool, phenylethyl alcohol and terpineol.

Often a single fragrance comprises a composition of various specifically combined scent components.

Essential oils mostly used as aroma components are preferably selected from the group consisting of bergamot oil, chamomile oil, rosemary oil, thyme oil, france kinsense oil, juniperberry oil, vetiver oil, lemon oil, lime oil, orange oil, mandarin oil, grapefruit oil, lavender oil, lemongrass oil, linden blossom oil, eucalyptus oil, melissa oil, myrtle oil, mint oil, pine needle oil, rose oil, sage oil, sandal wood oil, tea tree oil, olibanum oil, galbanum oil, labdanum oil, lavandin oil, ylang ylang oil and mixtures thereof.

Preference is also given to using bergamot oil, dihydromyrcenol, lilial, lyral, citronellol, phenylethyl alcohol, α-hexylcinnamaldehyde, geraniol, benzyl acetone, cyclamenaldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, vertofix coeur, evernyl, iraldein gamma, phenylacetic acid, geranyl acetate, benzyl acetate, rose oxide, romillat, irotyl and floramat alone or in mixtures.

A particularly preferred embodiment of the invention relates to cosmetic and/or pharmaceutical formulations comprising 0.1 to 80% by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one perfume and/or perfume oil or essential oil and/or fragrance in an amount of 0.01 to 10 %, preferably 0.1 to 3 % by weight based on the formulation.

### UV-light protection filters

With the inventive hydrocarbon mixtures, sensorily light, cosmetic and/or pharmaceutical formulations are obtained, which is the case especially when the hydrocarbon mixtures are used together with UV light protection filters.

The invention therefore provides cosmetic and/or pharmaceutical formulations comprising 0.1 to 80% by weight of hydrocarbons which comprise linear C11 and linear C13 hydrocarbons, where the sum of the linear C11 and linear C13 hydrocarbons is greater than or equal to 60% by weight, based on the sum of the hydrocarbons, and at least one UV light protection filter. According to the invention, suitable UV light protection filters are room temperature liquid or crystalline organic substances (light protection filters) which are capable of absorbing ultraviolet rays and releasing the energy absorbed again in the form of longer-wave radiation, for example heat. UV filters may be oil-soluble or water-soluble. Examples of typical oil-soluble UV B filters or broad-spectrum UV A/B filters include:
- 3-benzylidenecamphor or 3-benzylidenenorcamphor (Mexoryl SDS 20) and derivatives thereof, e.g. 3 (4-methylbenzylidene)camphor as described in EP 0693471 B1
- 3-(4'-trimethylammonium)benzylidenebornan-2-one methylsulfate (Mexoryl SO)
- 3,3'-(1,4-phenylenedimethine)bis(7,7-dimethyl-2-oxobicyclo[2.2.1]heptane-1-methanesulfonic acid) and salts (Mexoryl SX)
- 3-(4'-sulfo)benzylidenebornan-2-one and salts (Mexoryl SL)
- polymer of N-{(2 and 4)-[2-oxoborn-3-ylidene)-methyl}benzyl]acrylamide (Mexoryl SW)
- 2-(2H-benzotriazol-2-yl)-4-methyl-6-(2-methyl-3-(1,3,3,3-tetramethyl-1-(trimethylsilyloxy)-disiloxanyl)propyl)phenol (Mexoryl SL)
- 4-aminobenzoic acid derivatives, preferably 2 ethylhexyl 4-(dimethylamino)benzoate, 2-octyl 4 (dimethylamino)benzoate and amyl 4-(dimethyl-amino)benzoate;
- esters of cinnamic acid, preferably 2-ethylhexyl 4 methoxycinnamate, propyl 4-methoxycinnamate, isoamyl 4-methoxycinnamate, 2-ethylhexyl 2-cyano-3,3-phenylcinnamate (octocrylene);
- esters of salicylic acid, preferably 2-ethylhexyl salicylate, 4-isopropylbenzyl salicylate, homo¬menthyl salicylate;
- derivatives of benzophenone, preferably 2-hydroxy-4-methoxybenzophenone, 2-hydroxy-4-methoxy-4'-methylbenzophenone, 2,2'-dihydroxy-4-methoxybenzo¬phenone;
- esters of benzalmalonic acid, preferably di-2-ethylhexyl 4-methoxybenzmalonate;
- triazine derivatives, for example 2,4,6-trianilino(p-carbo-2'-ethyl-1'-hexyloxy)-1,3,5-triazine and 2,4,6-tris[p-(2-ethylhexyloxy-carbonyl)anilino]-1,3,5-triazine (Uvinul T 150), as described in EP 0818450 A1 or bis(2-ethylhexyl) 4,4'-[(6-[4-((1,1-dimethylethyl)aminocarbonyl)¬phenylamino]-1,3,5-triazine-2,4-diyl)diimino]¬benzoate (Uvasorb^{®} HEB);
- 2,2-(methylenebis(6-(2H-benzotriazol-2-yl)-4-(1,1,3,3-tetramethylbutyl)phenol) (Tinosorb M);
- 2,4-bis[4-(2-ethylhexyloxy)-2-hydroxyphenyl]-6-(4-methoxyphenyl)-1,3,5-triazine (Tinosorb S);
- propane-1,3-diones, for example 1-(4-tert-butylphenyl)-3-(4'-methoxyphenyl)propane-1,3-dione;
- ketotricyclo(5.2.1.0)decane derivatives, as described in EP 0694521 B1;
- dimethicodiethyl benzalmalonates (Parsol SLX).

- Useful water-soluble UV filters include:
- 2-phenylbenzimidazole-5-sulfonic acid and the alkali metal, alkaline earth metal, ammonium, alkylammonium, alkanolammonium and glucammonium salts thereof;
- 2,2-((1,4-phenylene)bis(1H-benzimidazole-4,6-disulfonic acid, monosodium salt) (Neo Heliopan AP);
- sulfonic acid derivatives of benzophenones, preferably 2-hydroxy-4-methoxybenzophenone-5-sulfonic acid and its salts;
- sulfonic acid derivatives of 3-benzylidenecamphor, for example 4-(2-oxo-3-bornylidenemethyl)benzene¬sulfonic acid and 2-methyl-5-(2-oxo-3-bornylidene)sulfonic acid and salts thereof.

Useful typical UV A filters are especially derivatives of benzoylmethane, for example 1-(4'-tert-butylphenyl)-3-(4'-methoxyphenyl)propane-1,3-dione, 4-tert-butyl-4'-methoxydibenzoylmethane (Parsol^{®} 1789), 1-phenyl-3-(4'-isopropylphenyl)propane-1,3-dione, and enamine compounds, as described in DE 19712033 A1 (BASF), and also benzoic acid 2-[4-(diethylamino)-2-hydroxy-benzoyl]hexyl ester (Uvinul^{®} A plus).

The UV A and UV B filters can of course also be used in mixtures. Particularly favorable combinations consist of the derivatives of benzoylmethane, e.g. 4-tert-butyl-4'-methoxydibenzoylmethane (Parsol^{®} 1789) and 2 ethylhexyl 2-cyano-3,3-phenylcinnamate (octocrylene) in combination with esters of cinnamic acid, preferably 2-ethylhexyl 4-methoxycinnamate and/or propyl 4 methoxycinnamate and/or isoamyl 4-methoxycinnamate. Combinations of this type are advantageously combined with water-soluble filters, for example 2 phenylbenzimidazole-5-sulfonic acid and the alkali metal, alkaline earth metal, ammonium, alkylammonium, alkanolammonium and glucammonium salts thereof.

Suitable UV light protection filters are especially the substances approved according to Annex VII of the Commission Directive (in the version Commission Directive 2005/9/EC of 28 January 2005 amending Council Directive 76/768/EEC, concerning cosmetic products, for the purposes of adapting Annexes VII thereof to technical progress), to which reference is hereby explicitly made.

In addition to the soluble substances mentioned, insoluble light protection pigments, specifically finely dispersed metal oxides and salts, are also useful for this purpose. Examples of suitable metal oxides are especially zinc oxide and titanium dioxide, and additionally oxides of iron, of zirconium, of silicon, of manganese, of aluminum and of cerium, and mixtures thereof. The salts used may be silicates (talc), barium sulfate or zinc stearate. The oxides and salts are used in the form of the pigments for skincare and skin-protecting emulsions, and also for decorative cosmetics. The particles should have a mean diameter of less than 100 nm, preferably between 5 and 50 nm and especially between 15 and 30 nm. They may have a spherical shape, but it is also possible to use those particles which have an ellipsoidal shape or a shape which deviates in some other way from the spherical configuration. The pigments may also be present in surface-treated form, i.e. hydrophilized or hydrophobized. Typical examples thereof are coated titanium dioxides, for example T 805 titanium dioxide (Degussa) or Eusolex^{®} T, Eusolex^{®} T-2000, Eusolex^{®} T Aqua, Eusolex^{®} AVO, Eusolex^{®} T-ECO, Eusolex^{®} T-OLEO and Eusolex^{®} T-S (Merck). Typical examples are zinc oxides, for example Zinc Oxide neutral, Zinc Oxide NDM (Symrise) or Z-Cote^{®} (BASF) or SUNZnO-AS and SUNZnO-NAS (Sunjun Chemical Co. Ltd.). Suitable hydrophobic coatings are in particular silicones and specifically trialkoxyoctylsilanes or simethicone. In sunscreen compositions, preference is given to using micropigments or nanopigments. Preference is given to using micronized zinc oxide. Further suitable UV light protection filters can be found in the review by P. Finkel in SÖFW Journal 122, 8/1996, pp. 543-548 and Parf. Kosm. 80th volume, no. 3/1999, p. 10 to 16.

In addition to the two aforementioned groups of primary light protection substances, it is also possible to use secondary light protection agents of the antioxidant type, which interrupt the photochemical reaction chain which is triggered when UV radiation penetrates into the skin. Typical examples thereof are amino acids (e.g. glycine, histidine, tyrosine, tryptophan) and derivatives thereof, imidazoles (e.g. urocanic acid) and derivatives thereof, peptides such as D,L carnosine, D-carnosine, L-carnosine and derivatives thereof (e.g. anserine), carotenoids, carotenes (e.g. α carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, linoleyl, cholesteryl and glyceryl esters thereof), and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (e.g. buthionine sulfoximines, homocysteine sulfoximine, buthionine sulfones, penta-, hexa-, heptathionine sulfoximine) in very low tolerated doses (e.g. pmol to mol/kg), also (metal) chelating agents (e.g. α-hydroxy fatty acids, palmitic acid, phytic acid, lactoferrin), α-hydroxy acids (e.g. citric acid, lactic acid, malic acid), humic acid, gallic acid, bile extracts, bilirubin, biliverdin, EDTA, EGTA and derivatives thereof, unsaturated fatty acids and derivatives thereof (e.g. gamma-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (vitamin A palmitate), and coniferyl benzoate of benzoin resin, rutinic acid and derivatives thereof, α-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, superoxide dismutase, zinc and derivatives thereof (e.g. ZnO, ZnSO4), selenium and derivatives thereof (e.g. selenomethionine), stilbenes and derivatives thereof (e.g. stilbene oxide, trans-stilbene oxide) and the derivatives (salts, esters, ethers, sugars, nucleotides, nucleosides, peptides and lipids), suitable in accordance with the invention, of these specified active ingredients.

A preferred embodiment of the invention relates to cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one UV light protection filter selected from the group consisting of 4-methylbenzylidenecamphor, benzophenone-3, butylmethoxydibenzoylmethane, bis-ethylhexyloxyphenol methoxyphenyl triazine, methylene bis-benzotriazolyl tetramethylbutylphenol, diethylhexyl butamido triazone, ethylhexyl triazone and diethylamino hydroxybenzoyl hexyl benzoate, 3-(4'-trimethylammonium)benzylidene¬bornan-2-one methylsulfate, 3,3'-(1,4-phenylenedimethine)bis(7,7-dimethyl-2-oxobicyclo[2.2.1]heptane-1-methanesulfonic acid) and its salts, 3-(4'-sulfo)¬benzylidenebornan-2-one and its salts, polymer of N-{(2 and 4)-[2-oxoborn-3-ylidene)methyl}benzyl]acrylamide, 2 (2H-benzotriazol-2-yl)-4-methyl-6-(2-methyl-3-(1,3,3, 3-tetramethyl-1-(trimethylsilyloxy)disiloxanyl)-propyl)phenol, dimethicodiethyl benzalmalonate and mixtures thereof.

These UV light protection filters are commercially available, for example, under the following trade names:
NeoHeliopan^{®}MBC (INCI: 4-Methylbenzylidene Camphor; manufacturer: Symrise); NeoHeliopan^{®}BB (INCI: Benzophenone-3, manufacturer: Symrise); Parsol^{®}1789 (INCI: Butyl Methoxydibenzoylmethane, manufacturer: Hoffmann-La Roche (Givaudan)); Tinosorb^{®}S (INCI: Bis-Ethylhexyloxyphenol Methoxyphenyl Triazine); Tinosorb^{®}M (INCI: Methylene Bis-Benzotriazolyl Tetramethylbutyl-phenol): manufacturer: Ciba Specialty Chemicals Corporation; Uvasorb^{®}HEB (INCI: Diethylhexyl Butamido Triazone, manufacturer: 3V Inc.), Uvinul^{®}T 150 (INCI: Ethylhexyl Triazone, manufacturer: BASF AG); Uvinul^{®} A plus (INCI: Diethylamino Hydroxybenzoyl Hexyl Benzoate: manufacturer: BASF AG); Mexoryl^{®} SO: 3-(4'-trimethylammonium)benzylidenebornan-2-one methylsulfate, INCI: Camphor Benzalkonium Methosulfate; Mexoryl^{®}SX: 3,3'-(1,4-phenylenedimethine)bis(7,7-dimethyl-2-oxobicyclo[2.2.1]heptane-1-methanesulfonic acid), CTFA: INCI Terephthalylidene Dicamphor Sulfonic Acid; Mexoryl^{®} SL: 3-(4'-sulfo)benzylidenebornan-2-one, INCI Benzylidene Camphor Sulfonic Acid; Mexoryl^{®}SW: polymer of N-{(2 and 4)-[2-oxoborn-3-ylidene)methyl}-benzyl]acrylamide, INCI Polyacrylamidomethyl Benzylidene Camphor; Mexoryl^{®}SL: 2-(2H-benzotriazol-2-yl)-4-methyl-6-(2-methyl-3-(1,3,3,3-tetramethyl-1-(trimethylsilyloxy)disiloxanyl)propyl)phenol; INCI: DROMETRIZOLE TRISILOXANE; Parsol^{®} SLX: dimethicodiethyl benzalmalonate, INCI Polysilicone-15.

The inventive formulations may comprise the UV light protection filters in amounts of 0.5 to 30% by weight, preferably 2.5 to 20% by weight, more preferably 5-15% by weight - based on the total weight of the cosmetic and/or pharmaceutical formulation.

The invention provides cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one self-tanning agent.

Self-tanning agents are understood to mean substances which cause browning of the skin. Examples include dihydroxyacetone, erythrulose and alpha, beta-unsaturated aldehydes, which react with the amino acids in the skin in the manner of a Maillard reaction to give colored compounds. Useful active ingredients for self-tanning agents also include natural or synthetic ketols or aldols. Examples of suitable active ingredients include dihydroxyacetone, erythrulose, glycerolaldehyde, alloxan, hydroxymethylglyoxal, gamma-dialdehyde, 6-aldo-D-fructose, ninhydrin and meso-tartaraldehyde. Suitable self-tanning agents are especially dihydroxyacetone and/or erythrulose.

Mixtures of the abovementioned active ingredients with one another or with muconaldehyde and/or naphthoquinones, for example 5-hydroxy-1,4-naphthoquinone (juglone) and 2-hydroxy-1,4-naphthoquinone, have been found to be particularly advantageous.

The inventive formulations comprise the self-tanning agents typically in concentrations of 1 to 10% and especially of 2 to 5% by weight - based on the total weight of the cosmetic and/or pharmaceutical formulation.

A particularly preferred embodiment of the invention relates to cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one UV light protection filter and at least one self-tanning agent.

The inventive cosmetic and/or pharmaceutical formulations may be present, for example, as O/W or W/O care emulsions, sunscreen formulations, antiperspirant/deodorant concepts, formulations for decorative cosmetics, oily care formulations, impregnation liquids for substrates, for example paper and nonwoven products. Examples include wet wipes, tissues, diapers or hygiene products.

The inventive hydrocarbon mixtures and the inventive cosmetic and/or pharmaceutical formulations are especially also suitable for light, sprayable applications and/or as constituents of care emulsions for tissues, papers, wipes, sponges (e.g. polyurethane sponges), plasters in the baby hygiene sector, babycare, skincare, sun protection, aftersun treatment, insect repellency, cleansing, face cleansing and antiperspirant/deodorant applications. They can be applied to tissues, papers, wipes, nonwoven products, sponges, puffs, plasters and bandages which find use in the cleansing, hygiene and/or care sectors (wet wipes for baby hygiene and babycare, cleansing wipes, face cleansing wipes, skincare wipes, care wipes with active ingredients to counteract skin aging, wipes with sunscreen formulations and insect repellents, and wipes for decorative cosmetics or for aftersun treatment, toilet wet wipes, antiperspirant wipes, diapers, tissues, wet wipes, hygiene products, self-tanning wipes, toilet paper, refreshing wipes, aftershave wipes). They can also be used, inter alia, in formulations for hair care, hair cleaning or hair coloring. The use of the inventive hydrocarbon mixtures positively influences the sensory performance on application. The inventive hydrocarbon mixtures are suitable especially as constituents of **decorative cosmetics formulations,** for example lipsticks, eye makeup, for example eyeshadow, mascara, eye pencils, kohl, nail varnish, etc., and makeup formulations.

The invention therefore provides cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one pigment and/or dye.

In addition the invention relates to a cosmetic composition of colored makeup and/or for the care of the skin or the lips comprising, at least 0.1% by weight of pigments and/or dyes with respect to the total weight of the composition and comprising 1 to 80% by weight with respect to the total weight of the composition, preferably 3 to 50% by weight, more preferably 5 to 25 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture.

### Pigments and Dyes

The term pigment encompasses particles of any kind which are white or colored, organic or inorganic, are insoluble in the formulations, and serve the purpose of coloring the formulation. The pigments may be present in a proportion of 0.1 to 15% by weight, especially 1 to 10% by weight, and in particular from 2 to 8% by weight, relative to the total weight of the cosmetic composition.

In a preferred embodiment, inorganic pigments are used, particular preference being given to metal oxides.

Examples of inorganic pigments include: titanium dioxide, optionally surface-coated, zirconium or cerium oxides, and zinc, iron (black, yellow or red) and chromium oxides, manganese violet, ultramarine blue, Prussian blue, chromium hydrates and iron(III) blue, metal powders such as aluminum powder or copper powder.
In a preferred embodiment of the invention, the pigment is selected from the inorganic pigments, preferably from the metal oxides. In a preferred embodiment, the pigment is selected from the group consisting of titanium dioxide, zinc oxide, iron oxide and mixtures thereof.

The pigments may be present either individually or in mixtures.

Preference is given in the context of the present invention to pigment mixtures composed of white pigments (e.g. kaolin, titanium dioxide or zinc oxide) and inorganic color pigments (e.g. iron oxide pigments, chromium oxides), and the pigments may be present in coated or uncoated form. Among the color pigments, iron oxides are particularly preferred. Advantageously in the context of the present invention, the pigment(s) may also be selected from the group of the effect pigments which impart to the cosmetic formulation, in addition to the pure color, an additional property - for example angular dependence of the color (flop), luster (not surface luster) or texture. Such effect pigments are used in accordance with the invention advantageously in addition to one or more white and/or color pigments.

The most important group of the effect pigments is that of the luster pigments, which, according to DIN 55944: 2003-11, include the metal effect pigments and the pearlescent pigments. Some specific effect pigments cannot be assigned to these two groups, for example graphite platelets, iron oxide platelets and micronized titanium dioxide, the latter not giving a luster effect, but rather an angle-dependent light-scattering effect. The luster pigments to DIN 55943: 2001-10 are predominantly effect pigment platelets. Aligned in parallel, luster pigments exhibit a characteristic luster. The visual effect of luster pigments is based on the directed reflection on metallic particles (metal effect pigments), on transparent particles with a high refractive index (pearlescent pigments) or on the phenomenon of interference (interference pigments) (DIN 55944: 2003 11).

Examples of commercial effect pigments preferred in accordance with the invention are: Timiron and #174; from Merck, Iriodin and #174; from Merck (pearlescent and color luster pigments for decorative industrial applications), Xirallic and #174; from Merck (intense-color crystal effect pigments).

In addition, the inventive formulations may advantageously also comprise organic color pigments, i.e. organic dyes which are virtually insoluble in the formulation. According to DIN 55944: 1990-04, organic pigments can be divided according to chemical aspects into azo pigments and polycyclic pigments, and according to color aspects into chromatic or black pigments. Organic white pigments are of no practical significance.

In the context of the present invention, the pigments can advantageously also be employed in the form of commercially available oily or aqueous pre-dispersions. The inventive formulations comprise typically 0.1 to 40% by weight of pigments - based on the total weight of the cosmetic and/or pharmaceutical formulation.

It is also advantageous in the context of the present invention when the inventive formulation comprises one or more dyes.
The dyes may be either of synthetic or natural origin.

A list of suitable dyes can be found in EP 1 371 359 A2, page 8, lines 25-57, page 9 and page 10, and also page 11, lines 1 to 54, to which reference is hereby explicitly made.
The inventive formulations comprise typically 0.01 to 5% and preferably 0.1 to 1.0% by weight of dyes - based on the total weight of the cosmetic and/or pharmaceutical formulation. The inventive formulations typically comprise a total amount of dyes and pigments in the range from 0.01 to 30% by weight, especially 0.1 to 15% by weight, preferably 1 to 10% by weight, based on the total weight of the cosmetic and/or pharmaceutical formulation.

Suitable dyes and pigments are especially the dyes and pigments approved according to Annex IV of the Commission Directive (in the version: Commission Directive 2007/22/EC of 17 April 2007 amending Council Directive 76/768/EEC, concerning cosmetic products, for the purposes of adapting Annexes IV and VI thereto to technical progress), to which reference is hereby explicitly made.

The cosmetic and/or pharmaceutical formulations may be formulations for bodycare, for example a body milk, creams, lotions, sprayable emulsions, products for eliminating body odor, etc. The hydrocarbon mixtures can also be used in surfactant-containing formulations, for example shower and bath gels, shampoos and care rinses as well as, eaux de toilette, eaux de parfum, eaux fraîches of, elixirs or fragrance extracts, aftershave lotions, of care water, biphasic lotions.

According to the end application, the cosmetic and/or pharmaceutical formulations comprise a series of further assistants and additives, for example surfactants, further oil bodies, emulsifiers, pearlescent waxes, bodying agents, thickeners, superfatting agents, stabilizers, polymers, fats, waxes, lecithins, phospholipids, biogenic active ingredients, antidandruff agents, film formers, swelling agents, insect repellents, self-tanning agents, tyrosinase inhibitors (depigmenting agents), hydrotropes, solubilizers, preservatives, perfume oils, dyes, etc., which are listed below by way of example.

The invention further provides cosmetic and/or pharmaceutical formulations comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one emulsifier and/or a surfactant and/or a wax component and/or a polymer and/or a further oil body.

### Antiperspirants

Antiperspirants are salts of aluminum, of zirconium or of zinc. Such suitable antihydrotic active ingredients are, for example, aluminum chloride, aluminum chlorohydrate, aluminum dichlorohydrate, aluminum sesquichlorohydrate and complexes thereof, for example with 1,2-propylene glycol, aluminum hydroxyallantoinate, aluminum chloride tartrate, aluminum zirconium trichlorohydrate, aluminum zirconium tetrachlorohydrate, aluminum zirconium pentachlorohydrate and complexes thereof, for example with amino acids such as glycine. Preference is given to using aluminum chlorohydrate, aluminum zirconium tetrachlorohydrate, aluminum zirconium pentachlorohydrate and complexes thereof.

The inventive formulations may comprise the antiperspirants in amounts of 1 to 50%, preferably 5 to 30% and especially 8 to 25% by weight - based on the total weight of the cosmetic and/or pharmaceutical formulation.

### Esterase inhibitors

In the presence of perspiration in the underarm region, bacteria form extracellular enzymes - esterases, preferably proteases and/or lipases - which cleave esters present in the perspiration and thus release odorants. Suitable esterase inhibitors are preferably trialkyl citrates such as trimethyl citrate, tripropyl citrate, triisopropyl citrate, tributyl citrate and especially triethyl citrate (Hydagen^{®} CAT, Cognis GmbH, Dusseldorf/FRG). The substances inhibit enzyme activity and hence reduce odor formation. Further substances which are possible esterase inhibitors are sterol sulfates or phosphates, for example sulfates or phosphates of lanosterol, of cholesterol, of campesterol, of stigmasterol and of sitosterol, dicarboxylic acids and esters thereof, for example glutaric acid, monoethyl glutarate, diethyl glutarate, adipic acid, monoethyl adipate, diethyl adipate, malonic acid and diethyl malonate, hydroxycarboxylic acids and esters thereof, for example citric acid, malic acid, tartaric acid or diethyl tartrate, and zinc glycinate.

The inventive formulations may comprise the esterase inhibitors in amounts of 0.01 to 20%, preferably 0.1 to 10% and especially 0.3 to 5% by weight - based on the total weight of the cosmetic and/or pharmaceutical formulation.

### Bactericidal or bacteriostatic active ingredients

Typical examples of suitable bactericidal or bacteriostatic active ingredients are especially chitosan and phenoxyethanol. 5-Chloro-2-(2,4-dichlorophenoxy)phenol has also been found to be particularly effective, and is sold under the Irgasan^{®} brand by Ciba-Geigy, Basle, Switzerland. Suitable germicides are in principle all substances which act against Grampositive bacteria, for example 4 hydroxybenzoic acid and the salts and esters thereof, N-(4-chlorophenyl)-N'-(3,4-dichlorophenyl)urea, 2,4,4'-trichloro-2'-hydroxydiphenyl ether (triclosan), 4 chloro-3,5-dimethylphenol, 2,2'-methylenebis(6-bromo-4-chlorophenol), 3-methyl-4-(1-methylethyl)phenol, 2 benzyl-4-chlorophenol, 3-(4-chlorophenoxy)-1,2-propanediol, 3-iodo-2-propynylbutyl carbamate, chlorhexidine, 3,4,4'-trichlorocarbanilide (TTC), antibacterial odorants, thymol, thyme oil, eugenol, clove oil, menthol, mint oil, farnesol, phenoxyethanol, glyceryl monocaprate, glyceryl monocaprylate, glyceryl monolaurate (GML), diglyceryl monocaprate (DMC), N-alkylsalicylamides, for example n-octylsalicylamide or n-decylsalicylamide.

The inventive formulations may comprise the bactericidal or bacteriostatic active ingredients in amounts of 0.01 to 5% and preferably 0.1 to 2% by weight - based on the total weight of the cosmetic and/or pharmaceutical formulation.

### Perspiration-absorbing substances

Useful perspiration-absorbing substances include modified starches, for example Dry Flo Plus (from National Starch), silicates, talc and other substances of similar polymorphism, which appear suitable for absorption of perspiration. The inventive formulations may comprise the perspiration-absorbing substances in amounts of 0.1 to 30%, preferably 1 to 20% and especially 2 to 8% by weight - based on the total weight of the cosmetic and/or pharmaceutical formulation.

With the inventive hydrocarbon mixtures, light, stable cosmetic and/or pharmaceutical formulations are obtained, which is the case especially when the hydrocarbon mixtures are used together with antiperspirant/deodorant active ingredients.

According to the invention, suitable antiperspirant/deodorant active ingredients are all active ingredients which counteract, mask or eliminate body odors. Body odors arise as a result of the action of skin bacteria on apocrine perspiration, which forms unpleasant-smelling degradation products. Suitable antiperspirant/deodorant active ingredients are especially compounds selected from the group consisting of antiperspirants, esterase inhibitors, bactericidal or bacteriostatic active ingredients and/or perspiration-absorbing substances.

Preferably the cosmetic and/or pharmaceutical formulations comprising the inventive hydrocarbon mixture are comprising at least one polyol and/or emulsifier and/or a surfactant and/or a wax component and/or a polymer and/or a further oil body.

More preferably the polyols are selected from the group consisting of Glycerin, Propylene Glycol, 1, 3-Butylene Glycol, Dipropylene Glycol, Neopentyl Glycol, 1, 2-Pentanediol or 1, 2-Hexanediol.

### Emulsifier

In one embodiment of the invention, the inventive formulations comprise at least one emulsifier.

The invention therefore further provides cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one emulsifier.

The inventive formulations comprise the emulsifier(s) typically in an amount of 0 to 40% by weight, preferably 0.1 to 20% by weight, preferably 0.1 to 15% by weight and especially 0.1 to 10% by weight, based on the total weight of the formulation.

Every emulsifier is assigned a so-called HLB value (a dimensionless number between 0 and 20) which specifies whether there is a preference for water or oil solubility. Numbers below 9 indicate preferentially oil-soluble, hydrophobic emulsifiers, numbers above 11 water-soluble, hydrophilic emulsifiers. The HLB value says something about the equilibrium of the size and strength of the hydrophilic and lipophilic groups of an emulsifier. The HLB value of an emulsifier can also be calculated from increments, and the HLB increments for the different hydrophilic and hydrophobic groups from which a molecule is composed can be found in tabular works (e.g. H.P. Fiedler, Lexikon der Hilfsstoffe für Pharmazie, Kosmetik und angrenzende Gebiete [Lexicon of the Excipients for Pharmacy, Cosmetics and Related Fields], Editio Cantor Verlag, Aulendorf, 4th Ed. 1996) or manufacturer data. The solubility of the emulsifier in oil or water effectively determines the emulsion type and the number of phases. When the emulsifier has better solubility in water, an O/W emulsion is obtained. When the emulsifier, in contrast, has better solubility in the oil phase, a W/O emulsion arises under otherwise identical production conditions. Adjusting the hydrophilicity and lipophilicity of the emulsifier e.g. one oil phase, one water phase, two separated phases of oil and water or a bi-continuous emulsion are obtained.

In one embodiment of the invention, the inventive formulation comprises more than one emulsifier. Depending on the other components, the person skilled in the art uses customary emulsifier systems (for example emulsifier and coemulsifier).

More preferably the emulsifier is selected from polyglycerol fatty acid esters selected from the group consisting of Polyglyceryl-2 Isostearate, Polyglyceryl-2 Oleate, Polyglyceryl-3 Diisostearate, Polyglyceryl-6 Dicaprate, Polyglyceryl-10 Laurate, polyglyceryl-10 Myristate, Polyglyceryl-10 Stearate, Polyglyceryl-10 Distearate, Polyglyceryl-10 Oleate, Polyglyceryl-10 Dioleate, Polyglyceryl-10 Pentaoleate, Polyglyceryl-10 Isostearate, Polyglyceryl-10 Diisostearate, Polyglyceryl-10 Triisostearate, Polyglyceryl-20 Triisostearate and non-ionic emulsifiers selected from the group consisting of PEG-8 Glyceryl Isostearate, PEG-7 Glyceryl Cocoate, PEG-7 Caprylic/Capric Glycerides, PEG-20 Glyceryl Triisostearate, PEG-12 Laurate, Sorbeth-30 Tetraoleate, Sorbitan Oleate, Polyoxyethylene Sorbitan Trioleate, PEG-5 Laureth-5, PPG-1-PEG-9 Lauryl Glycol Ether, PEG-40 Hydrogenated Castor Oil, Laureth-7 Citrate, alkylpolyglucosides.

### Nonionic emulsifiers

The group of nonionic emulsifiers includes, for example:
(1) Addition products of 2 to 50 mol of ethylene oxide and/or 1 to 20 mol of propylene oxide onto linear fatty alcohols having 8 to 40 carbon atoms, onto fatty acids having 12 to 40 carbon atoms and onto alkylphenols having 8 to 15 carbon atoms in the alkyl group.
(2) C12-C18 fatty acid mono- and diesters of addition products of 1 to 50 mol of ethylene oxide onto glycerol.
(3) Sorbitan mono- and diesters of saturated and unsaturated fatty acids having 6 to 22 carbon atoms and ethylene oxide addition products thereof.
(4) Alkyl mono- and oligoglycosides having 8 to 22 carbon atoms in the alkyl radical and ethoxylated analogs thereof.
(5) Addition products of 7 to 60 mol of ethylene oxide onto castor oil and/or hydrogenated castor oil.
(6) Polyol and especially polyglyceryl esters, for example polyol poly-12-hydroxystearates, polyglyceryl polyricinoleate, polyglyceryl diiso¬stearate or polyglyceryl dimerate. Likewise suitable are mixtures of compounds of two or more of these substance classes.
(7) Addition products of 2 to 15 mol of ethylene oxide onto castor oil and/or hydrogenated castor oil.
(8) Partial esters based on linear, branched, unsaturated or saturated C6-C22-fatty acids, ricinoleic acid and 12-hydroxystearic acid and polyglycerol, pentaerythritol, dipentaerythritol, sugar alcohols (e.g. sorbitol), alkyl glucosides (e.g. methyl glucoside, butyl glucoside, lauryl glucoside) and polyglucosides (e.g. cellulose), or mixed esters, for example glyceryl stearate citrate and glyceryl stearate lactate.
(9) Polysiloxane-polyalkyl-polyether copolymers and corresponding derivatives.
(10) Mixed esters of pentaerythritol, fatty acids, citric acid and fatty alcohol and/or mixed esters of fatty acids having 6 to 22 carbon atoms, methylglucose and polyols, preferably glycerol or polyglycerol.

The addition products of ethylene oxide and/or of propylene oxide onto fatty alcohols, fatty acids, alkylphenols, glyceryl mono- and diesters and also sorbitan mono- and diesters of fatty acids and onto castor oil are known, commercially available products. These are homolog mixtures whose mean degree of alkoxylation corresponds to the ratio of the amounts of ethylene oxide and/or propylene oxide and substrate with which the addition reaction is carried out. Depending on the degree of ethoxylation, they are W/O or O/W emulsifiers. C12/18 fatty acid mono- and diesters of addition products of ethylene oxide onto glycerol are known as refatting agents for cosmetic formulations.

Mild emulsifiers which are particularly suitable in accordance with the invention are polyol poly-12-hydroxystearates and mixtures thereof, which are sold, for example, under the "Dehymuls^{®} PGPH" (W/O emulsifier) or "Eumulgin^{®} VL 75" (blend with Lauryl Glucosides in a weight ratio of 1:1, O/W emulsifier) or Dehymuls^{®} SBL (W/O emulsifier) brands by Cognis Deutschland GmbH. In this connection, reference may be made especially to European patent EP 766 661 B1. The polyol component of these emulsifiers may derive from substances which have at least two, preferably 3 to 12 and especially 3 to 8 hydroxyl groups and 2 to 12 carbon atoms.

Particularly preferred emulsifiers are, for example, Cetyl Dimethicone Copolyol (e.g. Abil EM-90), Polyglyceryl-2 Dipolyhydroxystearate (e.g. Dehymuls PGPH), Polyglyceryl-3 Diisostearate (e.g. Lameform TGI), Polyglyceryl-4 Isostearate (e.g. Isolan GI 34), Polyglyceryl-3 Oleate (e.g. Isolan GO 33), Diisostearoyl Polyglyceryl-3 Diisostearate (e.g. Isolan PDI), Polyglyceryl-3 Methylglucose Distearate (e.g. Tego Care 450), Polyglyceryl-3 Beeswax (e.g. Cera Bellina), Polyglyceryl-4 Caprate (e.g. Polyglycerol Caprate T2010/90), Polyglyceryl-3 Cetyl Ether (e.g. Chimexane NL), Polyglyceryl-3 Distearate (e.g. Cremophor GS 32) and Polyglyceryl Polyricinoleate (e.g. Admul WOL 1403), Glyceryl Oleate (e.g. Monomuls 90-O 18), Alkyl Glucoside (e.g. Plantacare 1200, Emulgade PL 68/50, Montanov 68, Tego Care CG 90, Tego Glucosid L 55), Methyl Glucose Isostearate (e.g. Tego Care IS), Methyl Glucose Sesquistearate (Tego Care PS), Sodium Cocoyl Hydrolyzed Wheat Protein (e.g. Gluadin WK), Potassium Cetyl Phosphate (e.g. Amphisol K, Crodafos CKP), Sodium Alkylsulfate (e.g. Lanette E), Sucrose Ester (e.g. Crodesta F-10, F-20, F-50, F-70, F-110, F-160, SL-40, Emulgade^{®} Sucro), ethoxylated and/or propoxylated fatty alcohols, fatty acids, castor oils and hydrogenated castor oils (e.g. Eumulgin B2, B2, B3, L, HRE 40, HRE 60, RO 40, Cremophor HRE 40, HRE 60, L, WO 7, Dehymuls HRE 7, Arlacel 989), PEG-30 Dipolyhydroxystearate (e.g. Arlacel P 135, Dehymuls LE), sorbitan esters, sorbitan esters ethoxylated and/or propoxylated, and mixtures thereof. A particularly effective mixture consists of Polyglyceryl-2 Dipolyhydroxystearate and Lauryl Glucoside and glycerol (e.g. Eumulgin VL 75). Also suitable are Polyglyceryl-4 Diisostearate/Polyhydroxystearate/Sebacate (Isolan^{®} GPS), Diisostearoyl Polyglyceryl-3 Diisostearate (e.g. Isolan PDI), alkali metal acylglutamates (e.g. Eumulgin SG).

Suitable lipophilic W/O emulsifiers are in principle emulsifiers with an HLB value of 1 to 8, which are summarized in numerous tabular works and are known to the person skilled in the art. Some of these emulsifiers are listed, for example, in Kirk-Othmer, "Encyclopedia of Chemical Technology", 3rd edition, 1979, volume 8, page 913. For ethoxylated products, the HLB value can also be calculated according to the following formula: HLB = (100 - L):5, where L is the weight fraction of the lipophilic groups, i.e. of the fatty alkyl or fatty acyl groups in percent by weight, in the ethylene oxide adducts.

Particularly advantageous from the group of W/O emulsifiers are partial esters of polyols, especially of C4-C6-polyols, for example partial esters of pentaerythritol or sugar esters, e.g. sucrose distearate, sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxy-stearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical-grade mixtures thereof. Also suitable as emulsifiers are addition products of 1 to 30 and preferably 5 to 10 mol of ethylene oxide onto the specified sorbitan esters.

Depending on the formulation, it may be advantageous to additionally use at least one emulsifier from the group of nonionic O/W emulsifiers (HLB value: 8-18) and/or solubilizers. These are, for example, the ethylene oxide adducts already mentioned in the introduction and having a correspondingly high degree of ethoxylation, e.g. 10-20 ethylene oxide units for O/W emulsifiers and 20-40 ethylene oxide units for solubilizers. According to the invention, Ceteareth-12 and PEG-20 Stearate are particularly advantageous as O/W emulsifiers. Preferentially suitable solubilizers are Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Lauryl Glycol Ether), and Eumulgin^{®} SML 20 (INCI: Polysorbate-20).

Nonionic emulsifiers from the group of alkyl oligoglycosides are particularly skin-friendly and therefore preferentially suitable as O/W emulsifiers. C8 C22-alkyl mono- and oligoglycosides, their preparation and their use are known from the prior art. Their preparation takes place especially by reacting glucose or oligosaccharides with primary alcohols having 8 to 22 carbon atoms. As regards the glycoside radical, either monoglycosides, in which a cyclic sugar radical is glycosidically bonded to the fatty alcohol, or oligomeric glycosides with a degree of oligomerization up to preferably about 8 are suitable. The degree of oligomerization here is a statistical average based on a homolog distribution customary for such technical-grade products. Products which are available under the name Plantacare^{®} comprise a glucosidically bonded C8-C16-alkyl group onto an oligoglucoside radical whose average degree of oligomerization is 1 to 2. The acylglucamides derived from glucamine are also suitable as nonionic emulsifiers. According to the invention, preference is given to a product which is sold under the name Emulgade^{®}PL 68/50 by Cognis Deutschland GmbH and is a 1:1 mixture of alkyl polyglucosides and fatty alcohols. According to the invention, it is also advantageously possible to use a mixture of Lauryl Glucoside, Polyglyceryl-2 Dipolyhydroxystearate, glycerol and water, which is commercially available under the name Eumulgin^{®} VL 75.

Also suitable as emulsifiers are substances such as lecithins and phospholipids. Examples of natural lecithins which may be mentioned are the cephalins, which are also referred to as phosphatidic acids and are derivatives of 1,2-diacyl-sn-glycerol-3-phosphoric acids. By contrast, phospholipids are usually understood to mean mono- and preferably diesters of phosphoric acid with glycerol (glycerol phosphates), which are generally included in the fats. In addition, sphingosines and sphingolipids are also suitable.

The emulsifiers present may, for example, be silicone emulsifiers. These may be selected, for example, from the group of alkylmethicone copolyols and/or alkyldimethicone copolyols, especially from the group of compounds which are characterized by the following chemical structure:
in which X and Y are each independently selected from the group of H (hydrogen) and the branched and unbranched alkyl groups, acyl groups and alkoxy groups having 1-24 carbon atoms, p is 0-200, q is 1-40, and r is 1-100.

One example of silicone emulsifiers to be used particularly advantageously within the context of the present invention is that of dimethicone copolyols, which are sold by Evonik Goldschmidt under the trade names AXIL^{®} B 8842, ABIL^{®} B 8843, ABIL^{®} B 8847, ABIL^{®} B 8851, ABIL^{®} B 8852, ABIL^{®} B 8863, ABIL^{®} B 8873 and ABIL^{®} B 88183. A further example of interface-active substances to be used particularly advantageously within the context of the present invention is that of cetyl PEG/PPG-10/1 dimethicone (cetyl dimethicone copolyol), which is sold by Evonik Goldschmidt under the trade name ABIL^{®} EM 90. A further example of interface-active substances to be used particularly advantageously within the context of the present invention is that of cyclomethicone dimethicone copolyol, which is sold by Evonik Goldschmidt under the trade name ABIL^{®} EM 97 and ABIL^{®} WE 09. In addition, the emulsifier lauryl PEG/PPG-18/18 methicone (laurylmethicone copolyol) has been found to be very particularly advantageous and is available under the trade name Dow Corning^{®} 5200 Formulation Aid from Dow Corning Ltd. A further advantageous silicone emulsifier is octyl dimethicone ethoxy glucoside from Wacker.

For an inventive water-in-silicone oil emulsion, all known emulsifiers used for this type of emulsion can be used. Water-in-silicone emulsifiers which are particularly preferred in accordance with the invention are cetyl PEG/PPG-10/1 dimethicone and lauryl PEG/PPG-18/18 methicone [e.g. ABIL^{®} EM 90 (Evonik Goldschmidt), DC5200 Formulation Aid (Dow Corning)] and any desired mixtures of the two emulsifiers.

### Surfactants

In one embodiment of the invention, the inventive formulations comprise at least one surfactant.

Surfactants are amphiphilic substances which can dissolve organic, nonpolar substances in water. They cause, as a result of their specific molecular structure with at least one hydrophilic and a hydrophobic molecular moiety, a lowering of the surface tension of the water, the wetting of the skin, the facilitation of soil removal and dissolution, easy rinseoff and - if desired - foam regulation.

Surfactants are typically understood to mean surface-active substances which have an HLB value of greater than 20.

The invention therefore further provides cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one surfactant.
The surface-active substances present may be anionic, nonionic, cationic and/or amphoteric or zwitterionic surfactants. In surfactant-containing cosmetic formulations, for example shower gels, foam baths, shampoos, etc., at least one anionic surfactant is preferably present.

The inventive formulations comprise the surfactant(s) typically in an amount of 0 to 40% by weight, preferably 0.05 to 30% by weight, especially 0.05 to 20% by weight, preferably 0.1 to 15% by weight and especially 0.1 to 10% by weight, based on the total weight of the formulation.

Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partially oxidized alk(en)yl oligoglycosides and glucuronic acid derivatives, fatty acid N-alkylglucamides, protein hydrolyzates (especially wheat-based vegetable products), polyol fatty acid esters, sugar esters, sorbitan esters, polysorbates and amine oxides. If the nonionic surfactants contain polyglycol ether chains, they may have a conventional homolog distribution, but preferably have a narrow homolog distribution.

Zwitterionic surfactants refer to those surface-active compounds which bear at least one quaternary ammonium group and at least one -COO(-) or -SO3(-) group in the molecule. Particularly suitable zwitterionic surfactants are the betaines, such as the N-alkyl-N,N-dimethylammonium glycinates, for example cocoalkyl dimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazoline having in each case 8 to 18 carbon atoms in the alkyl or acyl group, and also cocoacylaminoethyl hydroxyethylcarboxymethyl glycinate. A preferred zwitterionic surfactant is the fatty acid amide derivative known under the INCI name Cocamidopropyl Betaine.

Likewise suitable, especially as cosurfactants, are ampholytic surfactants. Ampholytic surfactants are understood to mean those surface-active compounds which, apart from a C8-C18-alkyl or acyl group in the molecule, contain at least one free amino group and at least one -COOH or -SO3H group and are capable of forming internal salts. Examples of suitable ampholytic surfactants are N-alkylglycines, N-alkylpropionic acids, N-alkylaminobutyric acids, N-alkylimino¬dipropionic acids, N-hydroxyethyl-N-alkylamidopropyl¬glycines, N-alkyltaurines, N-alkylsarcosines, 2-alkyl¬aminopropionic acids and alkylaminoacetic acids having in each case about 8 to 18 carbon atoms in the alkyl group. Particularly preferred ampholytic surfactants are N-cocoalkylaminopropionate, cocoacylaminoethyl-aminopropionate and C12-18-acylsarcosine.

Typical examples of amphoteric or zwitterionic surfactants are alkylbetaines, alkylamidobetaines, aminopropionates, aminoglycinates, imidazolinium betaines and sulfobetaines. The specified surfactants are exclusively known compounds. With regard to the structure and preparation of these substances, reference may be made to relevant review works in this field. Typical examples of particularly suitable mild, i.e. particularly skin-friendly, surfactants are fatty alcohol polyglycol ether sulfates, monoglyceride sulfates, mono- and/or dialkyl sulfosuccinates, fatty acid isethionates, fatty acid sarcosinates, fatty acid taurides, fatty acid glutamates, α-olefinsulfonates, ether carboxylic acids, alkyl oligoglucosides and/or mixtures thereof with alkyl oligoglucoside carboxylates, fatty acid glucamides, alkylamidobetaines, amphoacetals and/or protein fatty acid condensates, the latter preferably based on wheat proteins or salts thereof.

Anionic surfactants are characterized by a water-solubilizing, anionic group, for example a carboxylate, sulfate, sulfonate or phosphate group and a lipophilic radical. Skin-compatible anionic surfactants are known to the person skilled in the art in a large number from relevant handbooks and are commercially available. These are especially alkyl sulfates in the form of their alkali metal, ammonium or alkanolammonium salts, alkyl ether sulfates, alkyl ether carboxylates, acyl isethionates, acyl sarcosinates, acyltaurines with linear alkyl or acyl groups having 12 to 18 carbon atoms, and also sulfosuccinates and acyl glutamates in the form of their alkali metal or ammonium salts.

Typical examples of anionic surfactants are soaps, alkylbenzenesulfonates, alkanesulfonates, olefin-sulfonates, alkyl ether sulfonates, glycerol ether sulfonates, α-methyl ester sulfonates, sulfo fatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ethercarboxylic acids and salts thereof, fatty acid isethionates, fatty acid sarcosinates, fatty acid taurides, N-acylamino acids, for example acyl lactylates, acyl tartrates, acyl glutamates and acyl aspartates, alkyl oligoglucoside sulfates, protein fatty acid condensates (especially vegetable products based on wheat) and alkyl (ether) phosphates. If the anionic surfactants comprise polyglycol ether chains, these may have a conventional homolog distribution, but preferably have a narrow homolog distribution.

Cationic surfactants which can be used are especially quaternary ammonium compounds. Preference is given to ammonium halides, especially chlorides and bromides, such as alkyltrimethylammonium chlorides, dialkyldimethyl¬ammonium chlorides and trialkylmethyl-ammonium chlorides, e.g. cetyltrimethylammonium chloride, stearyltrimethylammonium chloride, distearyldimethylammonium chloride, lauryldimethylammonium chloride, lauryldimethylbenzylammonium chloride and tricetylmethylammonium chloride. In addition, the very readily biodegradable quaternary ester compounds, for example the dialkylammonium methosulfates and methylhydroxyalkyldialkyloxyalkylammonium methosulfates sold under the trade name Stepantex^{®} and the corresponding products of the Dehyquart^{®} series can also be used as cationic surfactants. The term "ester quats" are generally understood to mean quaternized fatty acid triethanolamine ester salts. They can impart an exceptional soft feel to the preparations according to the invention. These are known substances which are prepared by the relevant methods of organic chemistry. Further cationic surfactants which can be used in accordance with the invention are the quaternized protein hydrolyzates.

### Wax component

In one embodiment of the invention, the inventive formulations comprise at least one wax component.

The invention therefore further relates to cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one wax component.

The inventive formulations comprise the wax component(s) typically in an amount of 0 to 40% by weight, especially of 0 to 20% by weight, preferably 0.1 to 15% by weight and especially 0.1 to 10% by weight, based on the total weight of the formulation.

The term "wax" is typically understood to mean all natural or synthetic substances and substance mixtures having the following properties: they are of solid to brittle and hard consistency, coarse to finely crystalline, transparent to cloudy and melt above 30°C without decomposition. They are low in viscosity even a little above the melting point and do not string, and exhibit a strongly temperature-dependent consistency and solubility. According to the invention, it is possible to use a wax component or a mixture of wax components which melt at 30°C or higher.

The waxes used in accordance with the invention may also be fats and fat-like substances with waxy consistency, provided they have the required melting point. These include, inter alia, fats (triglycerides), mono- and diglycerides, natural and synthetic waxes, fatty and wax alcohols, fatty acids, esters of fatty alcohols and fatty acids and also fatty acid amides or any desired mixtures of these substances.

Fats are understood to mean triacylglycerols, i.e. the triple esters of fatty acids with glycerol. They preferably comprise saturated, unbranched and unsubstituted fatty acid radicals. They may also be mixed esters, i.e. triple esters of glycerol with different fatty acids. According to the invention, it is possible to use hydrogenated fats and oils, which are obtained by partial hydrogenation and are particularly suitable as consistency regulators. Vegetable hydrogenated fats and oils are preferred, e.g. hydrogenated castor oil, peanut oil, soybean oil, rapeseed oil, colza oil, cottonseed oil, soybean oil, sunflower oil, palm oil, palm kernel oil, linseed oil, almond oil, corn oil, olive oil, sesame oil, cocoa butter and coconut fat.

Suitable examples include the triple esters of glycerol with C12-C60-fatty acids and especially C12-C36-fatty acids. These include hydrogenated castor oil, a triple ester of glycerol and a hydroxystearic acid, which is commercially available, for example, under the Cutina HR name. Glyceryl tristearate, glyceryl tribehenate (e.g. Syncrowax HRC), glyceryl tripalmitate or the triglyceride mixtures known under the Syncrowax HGLC name are likewise suitable, with the proviso that the melting point of the wax component or of the mixture is 30°C or higher.

According to the invention, usable wax components are especially mono- and diglycerides and mixtures of these partial glycerides. Glyceride mixtures which can be used in accordance with the invention include the Novata AB and Novata B (mixture of C12-C18-mono-, di- and triglycerides) and Cutina MD or Cutina GMS (glyceryl stearate) products sold by Cognis Deutschland GmbH & Co. KG.

Fatty alcohols which can be used in accordance with the invention as the wax component include the C12-C50-fatty alcohols. The fatty alcohols can be obtained from natural fats, oils and waxes, for example myristyl alcohol, 1-pentadecanol, cetyl alcohol, 1-heptadecanol, stearyl alcohol, 1-nonadecanol, arachidyl alcohol, 1 heneicosanol, behenyl alcohol, brassidyl alcohol, lignoceryl alcohol, ceryl alcohol or myricyl alcohol. Preference is given in accordance with the invention to saturated unbranched fatty alcohols. However, it is also possible in accordance with the invention to use unsaturated, branched or unbranched fatty alcohols as the wax component, provided they have the required melting point. It is also possible in accordance with the invention to use fatty alcohol cuts, as produced in the reduction of naturally occurring fats and oils, for example bovine tallow, peanut oil, colza oil, cottonseed oil, soybean oil, sunflower oil, palm kernel oil, linseed oil, castor oil, corn oil, rapeseed oil, sesame oil, cocoa butter and coconut fat. However, it is also possible to use synthetic alcohols, e.g. the linear, even-numbered fatty alcohols from the Ziegler synthesis (alfols) or the partially branched alcohols from the oxo process (dobanols). Particular preference is given in accordance with the invention to C14-C22-fatty alcohols, which are sold, for example, by Cognis Deutschland GmbH under the Lanette 18 (C18-alcohol), Lanette 16 (C16-alcohol), Lanette 14 (C14-alcohol), Lanette O (C16/C18-alcohol) and Lanette 22 (C18/C22-alcohol) names. Fatty alcohols impart a drier skinfeel to the formulations than triglycerides and are therefore preferred over the latter.

The wax components used may also be C14-C40-fatty acids or mixtures thereof. These include, for example, myristic acid, pentadecanoic acid, palmitic acid, margaric acid, stearic acid, nonadecanoic acid, arachic acid, behenic acid, lignoceric acid, cerotic acid, melissic acid, erucic acid and elaeostearic acid, and also substituted fatty acids, for example 12 hydroxystearic acid, and the amides or monoethanolamides of the fatty acids, this list being illustrative and nonlimiting in character.

It is possible in accordance with the invention to use, for example, natural vegetable waxes, such as candelilla wax, carnauba wax, japan wax, esparto grass wax, cork wax, guaruma wax, rice germ wax, sugarcane wax, ouricury wax, montan wax, sunflower wax, fruit waxes such as orange waxes, lemon waxes, grapefruit wax, bayberry wax, and animal waxes, for example beeswax, shellac wax, spermaceti, wool wax and uropygial grease. In the context of the invention, it may be advantageous to use hydrogenated or hardened waxes. The natural waxes which can be used in accordance with the invention also include mineral waxes, for example ceresin and ozokerite or the petrochemical waxes, for example petrolatum, paraffin waxes and microwaxes. Usable wax components also include chemically modified waxes, especially the hard waxes, for example montan ester waxes, sasol waxes and hydrogenated jojoba waxes. Synthetic waxes which can be used in accordance with the invention include, for example, wax-like polyalkylene waxes and polyethylene glycol waxes. Vegetable waxes are preferred in accordance with the invention.

The wax component can likewise be selected from the group of the wax esters of saturated and/or unsaturated, branched and/or unbranched alkanecarboxylic acids and saturated and/or unsaturated, branched and/or unbranched alcohols, from the group of esters of aromatic carboxylic acids, dicarboxylic acids, tricarboxylic acids and hydroxycarboxylic acids (e.g. 12-hydroxystearic acid) and saturated and/or unsaturated, branched and/or unbranched alcohols, and also from the group of lactides of long-chain hydroxycarboxylic acids. Examples of such esters are the C16-C40-alkyl stearates, C20-C40-alkyl stearates (e.g. Kesterwachs K82H), C20-C40-dialkyl esters of dimeric acids, C18-C38-alkylhydroxystearoyl stearates or C20-C40-alkyl erucates. It is also possible to use C30-C50-alkylbeeswax, tristearyl citrate, triisostearyl citrate, stearyl heptanoate, stearyl octanoate, trilauryl citrate, ethylene glycol dipalmitate, ethylene glycol distearate, ethylene glycol di(12-hydroxystearate), stearyl stearate, palmityl stearate, stearyl behenate, cetyl ester, cetearyl behenate and behenyl behenate. Fatty acid partial glycerides, i.e. technical-grade mono- and/or diesters of glycerol with fatty acids having 12 to 18 carbon atoms, for example glycerol mono/dilaurate, - palmitate, -myristate or stearate, are also useful for this purpose.

Suitable waxes are additionally pearlescent waxes. Useful pearlescent waxes, especially for use in surface-active formulations, are, for example: alkylene glycol esters, especially ethylene glycol distearate; fatty acid alkanolamides, especially coconut fatty acid diethanolamide; partial glycerides, especially stearic acid monoglyceride; esters of polybasic, optionally hydroxy-substituted carboxylic acids with fatty alcohols having 6 to 22 carbon atoms, especially long-chain esters of tartaric acid; fatty substances, for example fatty alcohols, fatty ketones, fatty aldehydes, fatty ethers and fatty carbonates, which have a total of at least 24 carbon atoms, especially laurone and distearyl ethers; fatty acids such as stearic acid, hydroxystearic acid or behenic acid, ring-opening products of olefin epoxides having 12 to 22 carbon atoms with fatty alcohols having 12 to 22 carbon atoms and/or polyols having 2 to 15 carbon atoms and 2 to 10 hydroxyl groups, and mixtures thereof.

### Polymers

In one embodiment of the invention, the inventive formulations comprise at least one polymer. The invention therefore further provides cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one polymer.

The inventive formulations comprise the polymer(s) typically in an amount of 0 to 20% by weight, preferably 0.1 to 15% by weight and especially 0.1 to 10% by weight, based on the total weight of the formulation.

Suitable cationic polymers are, for example, cationic cellulose derivatives, for example a quaternized hydroxyethylcellulose, which is available under the Polymer JR 400^{®} name from Amerchol, cationic starch, copolymers of diallylammonium salts and acrylamides, quaternized vinylpyrrolidone/vinylimidazole polymers, for example Luviquat^{®} (BASF), condensation products of polyglycols and amines, quaternized collagen polypeptides, for example lauryldimonium hydroxypropyl hydrolyzed collagen (Lamequat^{®}L/Grünau), quaternized wheat polypeptides, polyethyleneimine, cationic silicone polymers, for example amidomethicones, copolymers of adipic acid and dimethylaminohydroxypropyldiethylenetriamine (Cartaretine^{®}/Sandoz), copolymers of acrylic acid with dimethyldiallylammonium chloride (Merquat^{®} 550/Chemviron), polyaminopolyamides, cationic chitin derivatives for example quaternized chitosan, optionally in microcrystalline distribution, condensation products of dihaloalkylene, for example dibromobutane with bisdialkylamines, for example bisdimethylamino-1,3-propane, cationic guar gum, for example Jaguar^{®} CBS, Jaguar^{®} C-17, Jaguar^{®} C-16 from Celanese, quaternized ammonium salt polymers, for example Mirapol^{®} A-15, Mirapol^{®} AD-1, Mirapol^{®} AZ-1 from Miranol.

Useful anionic, zwitterionic, amphoteric and nonionic polymers are, for example, vinyl acetate/crotonic acid copolymers, vinylpyrrolidone/vinyl acrylate copolymers, vinyl acetate/butyl maleate/isobornyl acrylate copolymers, methyl vinyl ether/maleic anhydride copolymers and esters thereof, uncrosslinked polyacrylic acids and polyacrylic acids crosslinked with polyols, acrylamidopropyltrimethylammonium chloride/acrylate copolymers, octylacrylamide/methyl methacrylate/tert-butylaminoethyl methacrylate/2-hydroxypropyl methacrylate copolymers, polyvinylpyrrol¬idone, vinylpyrrolidone/vinyl acetate copolymers, vinylpyrrolidone/dimethylaminoethyl methacrylate/vinyl¬caprolactam terpolymers and optionally derivatized cellulose ethers and silicones.

Likewise suitable polymers are polysaccharides, in particular xanthan gum, guar guar, agar agar, alginates and tyloses and also, for example, Aerosil grades (hydrophilic silicas), carboxymethylcellulose and hydroxyethylcellulose and hydroxypropylcellulose, poly¬vinyl alcohol, polyvinylpyrrolidone and bentonites, for example Bentone^{®} Gel VS-5PC (Rheox). Likewise suitable are quaternary polymers, for example with the INCI name Polyquaternium-37, which conform to the following general formula:
Alternatively, it is also possible to use other dialkylaminoalkyl (meth)acrylates and their ammonium salts obtainable by alkylation or protonation, or dialkylaminoalkyl(meth)acrylamides and their ammonium salts obtainable by alkylation or protonation. Particular preference is given to polymers comprising MAPTAC, APTAC, MADAME, ADAME, DMAEMA and TMAEMAC. Moreover, it is also possible to use copolymers with anionic, further cationic or uncharged monomers in accordance with the invention, in particular those which, as well as the specified alkylaminoalkyl (meth)acrylate or alkylaminoalkyl(meth)acrylamide monomers, additionally comprise (meth)acrylic acid and/or 2-acrylamido-2-methylpropanesulfonic acid and/or acrylamide and/or vinylpyrrolidone and/or alkyl (meth)¬acrylates. By way of example, mention may be made of those polymers with the INCI name Polyquaternium-11, Polyquaternium-13, Polyquaternium-14, Polyquaternium-15, Polyquaternium-28, Polyquaternium-32, Polyquaternium-43, Polyquaternium-47.

### Oil bodies

In one embodiment of the invention, the inventive formulations comprise at least one oil body. Typically, the inventive formulations comprise the hydrocarbon mixture as the oil body. In the embodiment specified here as preferred, the formulations thus comprise an oil body other than the inventive hydrocarbon mixture, also referred to as "further oil body".

The invention therefore further provides cosmetic and/or pharmaceutical formulations comprising 0.1 to 80% by weight, preferably 0.5 to 50% by weight, more preferably 5 to 25 % and most preferably 1 to 5 % by weight of a hydrocarbon mixture which is comprising at least 95 % by weight of a linear C13 hydrocarbon based on the sum of hydrocarbons in the hydrocarbon mixture, C11/C12-hydrocarbons in an amount of 0.05 to 1.5 % by weight based on the sum of the hydrocarbons in the hydrocarbon mixture and C14 to C17-hydrocarbons in an amount of 0.2 to 3.5 % by weight based on the sum of the hydrocarbons in the hydrocarbon mixture, and at least one (further) oil body.

The oil bodies (inventive hydrocarbon mixture plus further oil bodies) are typically present in a total amount of 0.1-90%, especially 0.1-80%, especially 0.5 to 70%, preferably 1 to 60%, especially 1 to 50%, especially 1 to 40%, preferably 5-25% and especially 5 15% by weight. The further oil bodies are typically present in an amount of 0.1 to 40% by weight, based on the total weight of the formulation.

Suitable further oil bodies are, for example, Guerbet alcohols based on fatty alcohols having 6 to 18, preferably 8 to 10, carbon atoms, and also further additional esters such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Additionally suitable are esters of C18 C38-alkylhydroxycarboxylic acids with linear or branched C6-C22-fatty alcohols, especially dioctyl malate, esters of linear and/or branched fatty acids with polyhydric alcohols (for example propylene glycol, dimerdiol or trimertriol), triglycerides based on C6 C10-fatty acids, liquid mono-/di-/triglyceride mixtures based on C6-C18-fatty acids, esters of C6-C22-fatty alcohols and/or Guerbet alcohols with aromatic carboxylic acids, especially benzoic acid, esters of C2-C12-dicarboxylic acids with polyols having 2 to 10 carbon atoms and 2 to 6 hydroxyl groups, vegetable oils, branched primary alcohols, substituted cyclohexanes, linear and branched C6-C22-fatty alcohol carbonates, for example dicaprylyl carbonate (Cetiol^{®} CC), Guerbet carbonates based on fatty alcohols having 6 to 18, preferably 8 to 10, carbon atoms, esters of benzoic acid with linear and/or branched C6-C22-alcohols (e.g. Finsolv^{®} TN), linear or branched, symmetrical or asymmetrical dialkyl ethers having 6 to 22 carbon atoms per alkyl group, for example dicaprylyl ether (Cetiol^{®} OE), ring-opening products of epoxidized fatty acid esters with polyols and hydrocarbons or mixtures thereof.

Useful further oil bodies are, for example, silicone oils. They may be present as cyclic and/or linear silicone oils. Silicone oils are high molecular weight synthetic polymeric compounds in which silicon atoms are joined via oxygen atoms in a chain-like and/or grid-like manner and the remaining valences of silicon are satisfied by hydrocarbon radicals (usually methyl, more rarely ethyl, propyl, phenyl groups etc.). Systematically, the silicone oils are referred to as polyorganosiloxanes. The methyl-substituted polyorgano¬siloxanes, which are the most important compounds of this group in terms of volume and are characterized by the following structural formula are also referred to as polydimethylsiloxane or dimethicone (INCI). Dimethicones come in various chain lengths and with various molecular weights.

Advantageous polyorganosiloxanes in the context of the present invention are, for example, dimethylpoly-siloxane [poly(dimethylsiloxane)], which are available, for example, under the Abil 10 to 10 000 trade names from Evonik Goldschmidt. Also advantageous are phenylmethylpolysiloxane (INCI: Phenyl Dimethicone, Phenyl Trimethicone), cyclic silicones (octamethyl¬cyclotetrasiloxane or decamethylcyclopentasiloxane), which are also referred to in accordance with INCI as Cyclomethicone, amino-modified silicones (INCI: Amodimethicone) and silicone waxes, e.g. polysiloxane-polyalkylene copolymers (INCI: Stearyl Dimethicone and Cetyl Dimethicone) and dialkoxydimethylpolysiloxanes (Stearoxy Dimethicone and Behenoxy Stearyl Dimethicone), which are available as various Abil wax grades from Evonik Goldschmidt. However, other silicone oils can also be used advantageously in the context of the present invention, for example cetyldimethicone, hexamethylcyclotrisiloxane, polydimethylsiloxane, poly(methylphenylsiloxane). Silicones which are particularly preferred in accordance with the invention are dimethicone and cyclomethicone.

The inventive formulations may further comprise biogenic active ingredients, insect repellents, tyrosinase inhibitors, preservatives, perfume oils, superfatting agents, stabilizers and/or hydrotropes.

Biogenic active ingredients are understood to mean, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example Aloe Vera, prunus extract, bambara nut extract and vitamin complexes.

Useful insect repellents include, for example, N,N dimethyl-m-toluamide, 1,2-pentanediol or ethyl 3 (N-n-butyl-N-acetylamino)propionate), which is sold under the Insect Repellent^{®} 3535 name by Merck KGaA, and butylacetylaminopropionates.

Useful tyrosine inhibitors which prevent the formation of melanine and find use in depigmenting agents include, for example, arbutin, ferulic acid, kojic acid, cumaric acid and ascorbic acid (vitamin C).
Suitable preservatives are, for example, phenoxyethanol, formaldehyde solution, parabens, pentanediol or sorbic acid, and the silver complexes known under the Surfacine^{®} name. Additionally suitable as preservatives are the 1,2-alkanediols having 5 to 8 carbon atoms, which are described in WO 07/048757.

Suitable preservatives are especially the substances approved according to Annex VI of the Commission Directive (in the version: Commission Directive 2007/22/EC of 17 April 2007 amending Council Directive 76/768/EEC, concerning cosmetic products, for the purposes of adapting Annexes IV and VI thereto to technical progress), to which reference is made here explicitly.

The stabilizers used may be metal salts of fatty acids, for example stearates or ricinoleates of magnesium, aluminum and/or zinc.

To improve the flow behavior, it is also possible to use hydrotropes, for example ethanol, isopropyl alcohol or polyols. Polyols which are useful here possess preferably 2 to 15 carbon atoms and at least two hydroxyl groups. The polyols may contain further functional groups, especially amino groups, or be modified with nitrogen.

### Examples

### Example 1 - Manufacturing

### Preparation example 1: Preparation of an inventive hydrocarbon mixture

### 1a) Preparation of tridecane from 1-tetradecanol

1000 g of 1-tetradecanol (4.7 mol; Lanette 14 from BASF) were initially charged in a stirrable pressure vessel with 10 g of a nickel catalyst (Ni-5249 P from Engelhard; Ni content = 63% by weight) and heated to 240°C. Subsequently, hydrogen was added via a sparging tube at a pressure of 20 bar over a period of 12 h, and the reaction gases were simultaneously discharged through a valve on the reactor lid. Thereafter, the product was cooled, discharged and filtered. This gave a final weight of 845 g of reaction product.

A GC analysis shows the following composition in weight % based on the composition: 89.0% tridecane, 2.3% tetradecane, 4.0% 1-tetradecanol.
This reaction product was fractionated in a distillation to give the pure tridecane as classified, and then deodorized with nitrogen. This gives a colorless, mobile and low-odor product.

Composition of the hydrocarbon mixture according to example 1:
Undecane: 0.05
Dodecane: 0.237
Tridecane: 97.9
Tetradecane: 0.6
Pentadecane: 0.65
C16 decane: 0.22
C17-decane: 0.07

### Example 2 - Investigations with Electronic Nose

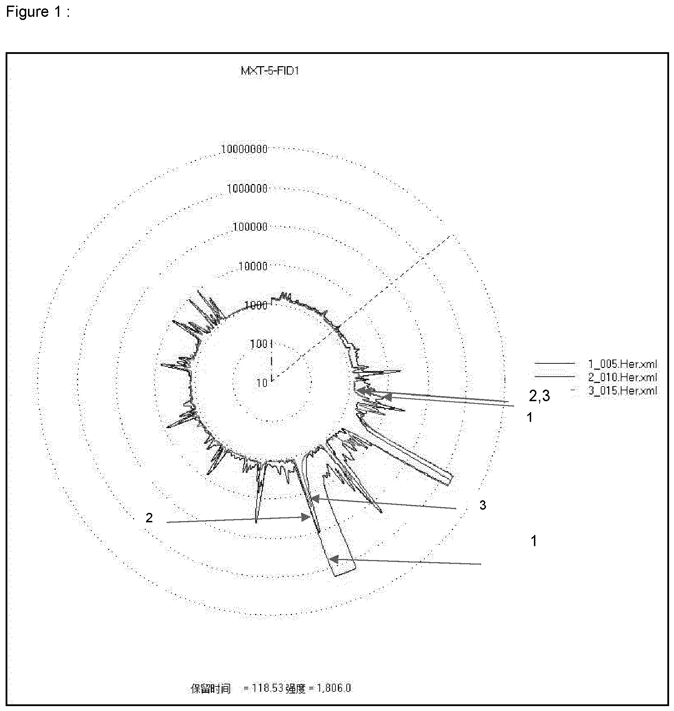

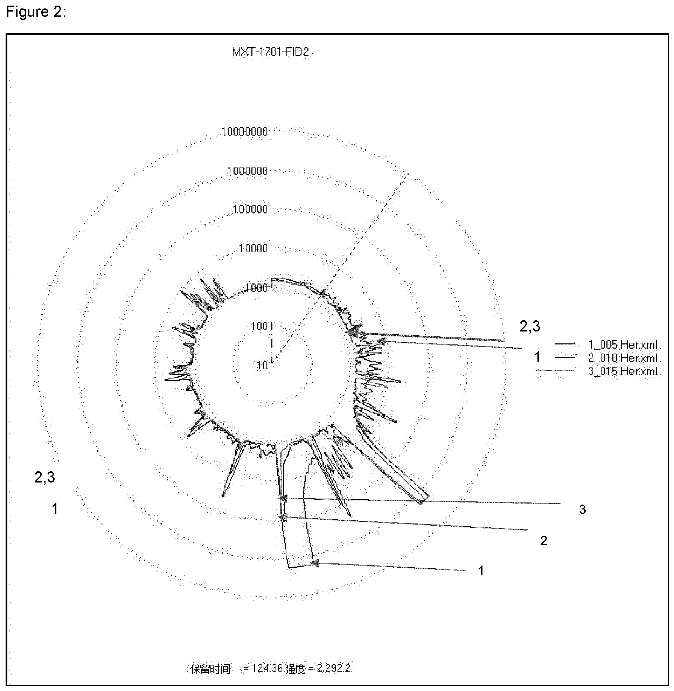

Three samples
- Hydrocarbon mixture according to example 1 (Cetiol iSAN, BASF) - radar graphs: figure 1a, 2a: blue line, figure 1b, 2b: arrow no. 2
- Undecane/tridecane (Cetiol Ultimate, BASF) -radar graphs: figure 1a, 2a: red line, figure 1b, 2b arrow no. 1
- Tridecane >99 % (Sigma Aldrich) - radar graphs: figure 1a, 2a: green line, figure 1b, 2b: arrow no. 3
have been investigated regarding their odor profile for non-polar components (figures 1a, 1b) and polar components (figures 2a, 2b) using an Electronic Nose Equipment.

The Electronic nose utilised in this comparison was FOX 4000 from Alpha-MOS (Toulouse, France), equipped with 18 metal oxide semiconductor gas sensors with a headspace autosampler HS100.

Two grams of the sample (the samples were analyzed in triplicate) were placed in a 10 mL volume of a vial and heated at 60 °C. 1 mL of headspace air was automatically injected into the electronic-nose by a syringe and sensor responses were recorded for 120 s (flushing with reference air). The maximum response points of electronic-nose, automatically recorded for each of 18 sensors, were used for analysis.

### Results:

### From the radar graphs

Figure 1 - Radarsheet electronic nose - non-polar
Figure 2 - Radarsheet electronic nose - polar
different profile of odor intensity could be observed. The sensor gave highest response to volatile compound of Cetiol Ultimate (red line), but relatively low for Cetiol iSan (blue line) and tridecane from Aldrich (>99%) (green line). The latter two gave close odor profiles.

### Example 3 - Sensority test

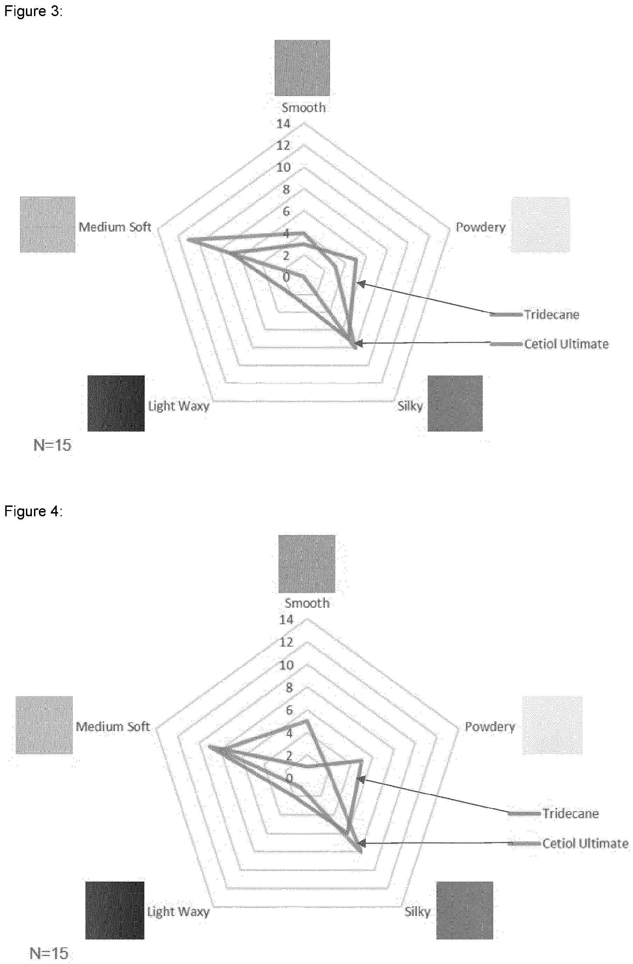

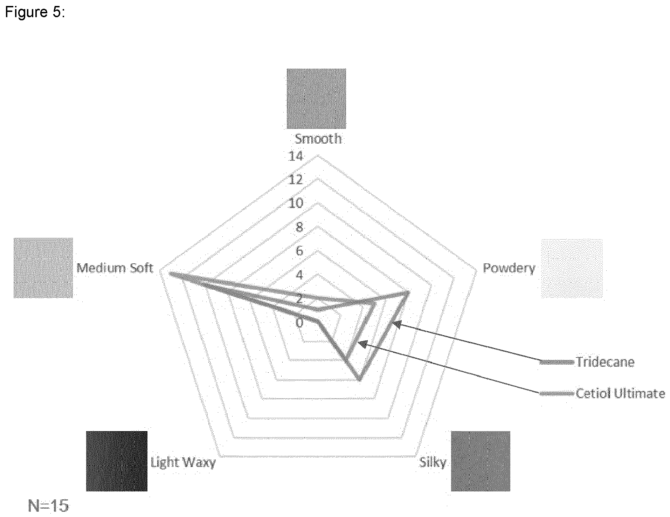

The hydrocarbon mixture according to example 1 was tested by a panel of 15 participants via an experiment named "pillow talk" and compared with Cetiol Ultimate.
- The participants first felt 5 different pillows with both hands with eyeshades to avoid bias from the appearance of the pillows. The participants need to feel the pillow by their own perception.
- 40 microliter od emollient product is then applied on the forearm of the participants and spread by 20 circles. Then the assessment of the product started immediately.
- The participants felt the 5 pillows again and select the pillow(s) which fit to the sensorial perception, so they chose the pillows which are close to the skin feel. It was possible to choose all the pillows.
- The same procedure is repeated again after 1 min and 3 min, and the pillow(s) are selected.
- Statistics: the selection of pillow was recorded. The number was presented in a spider web (see Figure 3 - Sensority assessment directly after application, Figure 4 - Sensority assessment after 3 minutes, Figure 5 - Sensority assessment after 5 minutes).

Figures 3 to 5 show that the application of the hydrocarbon mixture according to example 1 does not result in any waxy impression. Compared with Cetiol Ultimate (orange line of figures 3 to 5) the hydrocarbon mixture according to example 1 (blue line of figures 3 to 5) showed less medium soft feeling immediately after application. (figure 3). One minute after application the tridecane according to example 1 showed obviously more powdery but less smooth feeling than Cetiol Ultimate (figure 4) and three minutes after application compared to Cetiol Ultimate, it showed stronger powdery and silky, but less medium soft feeling (figure 5). However, the general sensorial profile of both hydrocarbon mixtures is comparable and it is surprising that the hydrocarbon mixture of the invention has the same non-waxy impression than the C11/C13-mixture with shorter hydrocarbon.

### Formulation examples:

### 4.1 "Deep Clean" Mild Cleansing oil

| **Phase** | **Ingredients** | **INCI** | **% by weight** | **Function** |
|---|---|---|---|---|
| A | Cetiol SN-1 | Cetyl Ethylhexanoate | 49.60 | Emollient |
| | Cetiol CC | Dicaprylyl Carbonate | 10 | Emollient |
| | Cetiol OE | Dicaprylyl Ether | 15 | Emollient |
| | Cetiol iSAN | Tridecane of example 1 | 10 | Emollient |
| | Vitamin E-Acetate Care | Tocopheryl Acetate | 0.40 | Antioxidant |
| B | Plantapon LC7 | Laureth-7 Citrate | 5.50 | Surfactant |
| | Lameform TGI | Polyglyceryl-3 Diisostearate | 4.50 | structurant |
| | Cetiol HE | PEG-7 Glyceryl Cocoate | 5 | Emollient |
| C | Neutrol TE | Tetrahydroxypropyl Ethylenediamine | q.s. | Neutralizing agent |
| pH value | | | 5.0-5.5 | |
| Appearance | | | | Clear, colourless to pate yellow liquid |

### 4.2 Water Bank

| Phase | Ingredients | INCI | % by weight | Function |
|---|---|---|---|---|
| A | Eumulgin SG | Sodium Stearoyl Glutamate | 0.05 | Emulsifier |
| | Cosmedia SP | Sodium Polyacrylate | 1 | Rheology modifier |
| | Cetiol LC | Coco-Caprylate/Caprate | 5 | Emollient |
| | Cetiol iSAN | Tridecane of example 1 | 5 | Emollient |
| B | Glycerin | Glycerin | 3 | Humectant |
| | Water | Aqua | 85.95 | |
| C | | | q.s. | Preservative |
| | Perfume | Parfum | q.s. | Fragrance |
| pH value (23°C) | | | 6.5 | |

### 4.3 Bi-continous Cleansing liquid

| **Phase** | **Ingredients** | **INCI** | **% by weight** | **Function** |
|---|---|---|---|---|
| A | Cetiol SN-1 | Cetyl Ethylhexanoate | 5 | Emollient |
| | Cetiol OE Deo | Dicaprylyl Ether | 4 | Emollient |
| | Cetiol iSAN | Tridecane of example 1 | 2 | Emollient |
| | Cetiol Sensoft | Propylheptyl Caprylate | 2 | Emollient |
| | Cetiol RLF Deo | Caprylyl caprylate/caprate | 2 | Emollient |
| | Eutanol G-JP | Octyldodecanol | 3 | Emollient |
| | Cetiol HE-JP | PEG-7 Glyceryl Cocoate | 10 | Solubilizer |
| | Eumulgin HPS | Coceth-7, PPG-1-PEG-9 Lauryl Glycol Ether, PEG-40 Hydrogenated Castor Oil | 10 | Solubilizer |
| | Lameform TGI | Polyglyceryl-3 Diisostearate | 6 | Co-Solubilizer |
| | Plantacare 2000 UP | Decyl Glucoside | 3 | Surfactant |
| | Plantapon LC7 | Laureth-7 Citrate | 2 | Surfactant |
| | Glycerin | Glycerin | 20 | Humectant |
| | Dipropylene Glycol Care | Dipropylene Glycol | 10 | Humectant |
| | Sodium Hydroxide (5% Lösung) | Sodium Hydroxide | 1 | pH-Adjustment |
| | Preservative | | q.s. | Preservative |
| Water | Aqua | | 20 | |
| pH Value | | | 5.0-6.0 | |

### 4.4 Oil in Awa Cleansing - Pump foam type

| **Phase** | **Ingredients** | **INCI** | **% by weight** | **Function** |
|---|---|---|---|---|
| A | Cetiol HE-JP | PEG-7 Glyceryl Cocoate | 6 | Solubilizer |
| | Cetiol iSAN | Tridecane of example 1 | 1 | Emollient |
| | Cetiol C 5C | Coco-Caprylate/caprate | 1 | Emollient |
| B | Plantacare 818 UP | Coco-Glucoside | 14 | Surfactant |
| | Plantapon LC7 | Laureth-7 Citrate | 4 | Surfactant |
| | Plantapon ACG HC | Sodium Cocoyl Glutamat | 8 | Surfactant |
| | Dehyton PK 45 | Cocamidopropyl Betaine | 3.30 | Surfactant |
| | Glycerin | Glycerin | 2 | Humectant |
| | Pluracare E 400 | PEG-8 | 5 | Solubilizer |
| | Pluracare F 127 NF Prill (10% aqueous solution) | Water, Poloxamer 407 | 10 | Foam booster |
| | Preservative | | q.s. | Preservative |
| | Water | Aqua | 45.70 | |
| C | Citric acid | Citric acid | q.s. | pH-Adjustment |
| | | | | |
| pH Value | | | 5.0-6.0 | |

### 4.5 Cleansing balm

| **Ingredients** | **INCI** | **% by weight** | **Function** |
|---|---|---|---|
| Cutina GMS V | Glyceryl Stearate | 15 | Consistency agent |
| Cutina HR Powder | Hydrogenated Castor Oil | 2 | Consistency agent |
| Micropoly 204 (Micro powders) | Synthetic wax | 5 | Consistency agent |
| Irwinol LS 9890 | Octyldodecanol, Irvingia Gabonensis, Hydrogenated Coco-Glycerides | 1 | Active Ingredient |
| Lipofructyl PI LS 9324 | Prunus Insititia Seed Oil | 2 | Active ingredient |
| Cetiol SN-1 | Cetyl Ethylhexanoate | 47 | Emollient |
| Myritol GTEH | Triethylhexanoin | 12 | Emollient |
| Cetiol PEEH-4 | Pentaerythrityl Tetraehtylhexanoate | 5 | Emollient |
| Cetiol RLF Deo | Caprylyl caprylate/caprate | 2 | Emollient |
| Cetiol OE Deo | Dicaprylyl Ether | 1 | Emollient |
| Cetiol iSAN | Tridecane of example 1 | 1 | Emollient |
| Cetiol HE-JP | PEG-7 Glyceryl Cocoate | 5 | Solubilizer |
| Cetiol HE 810 | PEG-7 Caprylic/capric Glycerides | 2 | Solubilizer |

### 4.6 UV Cut Splash - SPF 50, PA ++++ (in silica)

| **Phase** | **Ingredients** | **INCI** | **% by weight** | **Function** |
|---|---|---|---|---|
| A | Uvinul QD | Ethylhexyl Methoxycinnamate, Diethylamino Hydroxybenzoyl Hexyl Benzoate, Bis-Ethylhexyloxyphenol Methoxyphenyl Triazine | 10.80 | Broad spectrum UV filter |
| | Cetiol iSAN | Tridecane | 1.20 | Emollient |
| | Cetiol CC | Dicaprylyl Carbonate | 1.20 | Emollient |
| | Cetiol RLF Deo | Caprylyl caprylate/caprate | 0.80 | Emollient |
| | Myritol PGDC | Propylene Glycol Dicaprylate/Caprate | 0.40 | Emollient |
| | Lipofructyl Argan LS 9779 | Argania spinosa Kernel Oil | 0.20 | Active ingredient |
| | Silica | Silica | 0.40 | Skin feel modifier |
| | Ethanol | Alcohol | 25 | Solvent |
| B | LPG | Petroleum Distillates | 60 | Propellant |
| Ratio solution/propellant (aerosol) incl. packaging & filling (Aluminium; Mitsuya valve D94; Mitsuya valve S13; LPG; 2.7 bar) | | | 40:60 | |
| SPF in silico (BASF Sunscreen Simulator - in silico determination of | | | 50 | |
| the sun protection factor) | | | | |
| UV PF in silico (BASF Sunscreen Simulator - in silico determination of the PPD (UVA-PF in vivo)) | | | 17.40 | |

### 4.7 Babymilk Intense Care

| **Phase** | **Ingredients** | **INCI** | **% by weight** | **Function** |
|---|---|---|---|---|
| A | Dehymuls PGPH | Polyglyceryl-2 Dipolyhydroxystearate | 3.00 | Emulsifier (W/O) |
| | Monomuls 90-O 18 | Glyceryl Oleate | 1.00 | Emulsifier (W/O) |
| | Beeswax 8108 (Kahl) | Beeswax | 1.00 | Consistency Agent |
| | Zinc Stearate (Peter Greven) | Zinc Stearate | 1.00 | Stabilizer |
| | Sunflower Oil | Helianthus Annuus (Sunflower) Seed Oil | 5.00 | Emollient |
| | Cetiol RLF | Capylyl Caprylate/Caprate | 2.00 | Emollient |
| | Cetiol iSAN | Tridecane of example 1 | 3.00 | Emollient |
| | Cetiol 4 all | Dipropylheptyl Carbonate | 4.50 | Emollient |
| | Cetiol 868 | Ethylhexyl Stearate | 7.00 | Emollient |
| | Cetiol SB 45 | Butyrospermum Parkii (Shea) Butter | 0.50 | Emollient |
| | Copherol 1250 C | Tocopheryl Acetate | 0.20 | Active ingredient |

### 4.8 Baby Protective Ointment

| **Ingredients** | **INCI** | **% by weight** | **Function** |
|---|---|---|---|
| Dehymuls PGPH | Polyglyceryl-2 Dipolyhydroxystearate | 1.00 | Emulsifier (W/O) |
| Lameform TGI | Polyglyceryl-3 Diisostearate | 3.00 | Emulsifier (W/O) |
| Beeswax 8108 (Kahl) | Beeswax | 1.00 | Consistency Agent |
| Zinc Stearate (Peter Greven) | Zinc Stearate | 1.00 | Stabilizer |
| Zinc Oxide | Zinc oxide | 10.00 | Active ingredient |
| Sunflower Oil | Helianthus Annuus (Sunflower) Seed Oil | 5.00 | Emollient |
| Cetiol 868 | Ethylhexyl Stearate | 5.00 | Emollient |
| Cetiol 4 all | Dipropylheptyl Carbonate | 5.00 | Emollient |
| Cetiol iSAN | Tridecane of example 1 | 3.00 | Emollient |
| Phytosoothe LS 9766 | Brassica campestris (Rapeseed) Sterols, Cetearyl alcohol | 2.00 | Active ingredient |

### 4.9 Blue Lagoon Body Care

| **Phase** | **Ingredients** | **INCI** | **% by weight** | **Function** |
|---|---|---|---|---|
| A | Water, demin. | Aqua | 84.40 | |
| | Rheocare C plus | Carbomer | 0.70 | Rheology modifier |
| B | Glycerin | Glycerin | 6.00 | Humectant |
| | Rheocare XGN | Xanthan Gum | 0.50 | Rheology modifier |
| C | Cegesoft HF 52 | Hydrogenated vegetable oil | 0.75 | Consistency agent |
| | Cetiol SB 45 | Butyrospermum Parkii Butter | 1.25 | Emollient |
| D | Cegesoft Peel | Glycol Distearate | 1.00 | Exfoliant |
| | Puricolor Blue ABL9 FDA (1% solution) | CI 42090 | 0.10 | Colorant |
| E | Cetiol iSAN | Tridecane of example 1 | 1.50 | Emollient |
| | Plantacare 810 UP | Caprylyl/Capryl Glucoside | 1.50 | Surfactant |
| | Eumulgin SML 20 | Polysorbate 20 | 2.00 | Solubilizer |
| | Reflecks Pinpoints of Pearl G130L | Calcium Sodium Borosilicate, Titanium Dioxide | 0.05 | Effect pigment |
| | Preservative | | qs | Preservative |
| | Perfume | Parfum | 0,25 | Fragrance |
| F | Sodium Hydroxide (25% solution) | Sodium Hydroxide | qs | pH Adjustment |
| pH value 20°C | | | 5.9-6.1 | |

### 4.10 Bronzing Body Butter

| **Phase** | **Ingredients** | **INCI** | **% by weight** | **Function** |
|---|---|---|---|---|
| A | Water, demin. | Aqua | 42.15 | |
| | Vanatural | Bentonite | 2.00 | Rheology modifier |
| | Vanzan NF | Xanthan Gum | 0.50 | Stabilizer |
| | Eumulgin SG | Sodium Stearoyl Glutamate | 0.90 | Emulsifier |
| | Plantacare 818 UP | Coco-Glucoside | 0.30 | Emulsifier |
| | Hydrasensyl Glucan | Aqua, Beta-Glucan | 3.00 | Active Ingredient |
| | Potassium Sorbate | Potassium Sorbate | 0.30 | Preservative |
| B | Cutina GMS | Glyceryl Stearate | 6.40 | Consistency Agent |
| | Lanette O | Cetearyl Alcohol | 2.40 | Consistency Agent |
| | Cetiol CC | Dicaprylyl Carbonate | 2.00 | Emollient |
| | Lanette 22 | Behenyl Alcohol | 1.00 | Consistency Agent |
| | Cetiol OE | Dicaprylyl Ether | 3.00 | Emollient |
| | Cetiol iSAN | Tridecane of example 1 | 3.00 | Emollient |
| | Cetiol SB 45 | Butyrospermum Parkii Butter | 10.00 | Emollient |
| | Lipofructyl MO LS 9305 | Moringa Pterygosperma Seed Oil, Moringa oleifera seed oil | 2.00 | Active Ingredient |
| C | Benzyl Alcohol | Benzyl Alcohol | 1.00 | Preservative |
| D | Cloisonné Orange 363C | Mica, Titanium Dioxide, Iron Oxides | 5.00 | Effect Pigment |
| | Reflecks Dimensions Brilliant Gold GY80D | Calcium Sodium Borosilicate, Titanium Dioxide, Silica | 5.00 | Effect Pigment |
| | Water, demin. | Aqua | 10.00 | |
| E | Citric Acid (25% solution) | Citric acid | 0.05 | pH Adjustment |
| pH value 23°C | | | 6.0 - 7.0 | |

### 4.11 Eye Primer

| **Phase** | **Ingredients** | **INCI** | **% by weight** | **Function** |
|---|---|---|---|---|
| A | Eumulgin VL 75 | Lauryl Glucoside, Polyglyceryl-2 Dipolyhydroxystearate, Glycerin | 1.00 | Emulsifier |
| | Cetiol SB 45 | Butyrospermum Parkii Butter | 2.00 | Emollient |
| | Cetiol C5 | Coco-Caprylate | 3.00 | Emollient |
| | Cetiol Sensoft | Propylheptyl Caprylate | 2.00 | Emollient |
| | Cetiol iSAN | Tridecane of example 1 | 2.00 | Emollient |
| | KSG-210 | Dimethicone, Dimethicone/ PEG-10/15 Crosspolymer | 2.00 | Emulsifier |
| | Cosmedia SP | Sodium Polyacrylate | 0.80 | Rheology modifier |
| | Euxyl PE 9010 | Phenoxyethanol, Ethylhexylglycerin | 1.00 | Preservative |
| B | Water, demin. | Aqua | 77.80 | |
| | Glycerin | Glycerin | 5.00 | Humectant |
| | Rheocare C plus | Carbomer | 0.10 | Rheology modifier |
| | Edeta BD | Disodium EDTA | 0.05 | Complexing agent |
| | Sodium Hydroxide (18% solution) | Sodium Hydroxide | 0.50 | pH Adjustment |
| C | Covi-ox T 70 C | Tocopherol | 0.50 | Antioxidant |
| | Sqisandryl LS 9905 | Schisandra Chinensis Fruit Extract | 0.30 | Active Ingredient |
| D | Dry-Flo PC | Aluminum Starch Octenylsuccinate | 0.40 | Skin feel modifier |
| | Gemtone Tan Opal G005 | Mica, Iron Oxides, Titanium Dioxide | 0.50 | Effect pigment |
| | Chione HD Digital Ping S430V | Synthetic Fluorphlogopite, Titanium Dioxide | 0.83 | Effect pigment |
| | Sodium Hydroxide | Sodium Hydroxide | 0.22 | pH Adjustment |
| pH value 23°C | | | 6.2 | |

### 4.12 Perfume containing Emulsion

| **Phase** | **Ingredients** | **INCI** | **% by weight** | **Function** |
|---|---|---|---|---|
| A | Emulgade Sucro Plus | Sucrose Polystearate, Cetyl Palmitate | 1.00 | Emulsifier (O/W) |
| | Lanette O | Cetearyl Alcohol | 4.00 | Consistency agent |
| | Cetiol LC | Coco-Caprylate/Caprate | 6.00 | Emollient |
| | Cetiol Sensoft | Propylheptyl Caprylate | 2.00 | Emollient |
| | Cetiol 4 all | Dipropylheptyl Carbonate | 1.00 | Emollient |
| | Cetiol iSAN | Tridecane of example 1 | 3.00 | Emollient |
| B | Water, demin. | Aqua | 65.77 | |
| | Euxyl PE 9010 | Phenoxyethanol, Ethylhexylglycerin | 1.10 | Preservative |
| | Glycerin | Glycerin | 3.50 | Humectant |
| | Sodium Hydroxide (18% solution) | Sodium Hydroxide | 0.25 | pH Adjustment |
| C | Rheocare C plus | Carbomer | 0.20 | Rheology modifier |
| | Water, demin. | Aqua | 10.00 | |
| D | Puricolor Red ARE 33 FDA (1% solution) | CI 17200 | 0.01 | Colorant |
| E | Perfume | Parfum | 0.17 | Fragrance |
| | Sacred Patch BC 10022 | Aqua, Glycerin, Pentylene Glycol, Algin, Caprylyl Glycol, Glyceryl Polyacrylate, Sodium Hyaluronate, Pullulan, Aphanothece Sacrum Polysaccharides | 2.00 | Active Ingredient |
| pH value 23°C | | | 6.0 | |

### 4.13 Length Protection Lotion

| **Phase** | **Ingredients** | **INCI** | **% by weight** | **Function** |
|---|---|---|---|---|
| A | Water, demin. | Aqua | 84.40 | |
| | Dehyquart A-CA | Cetrimonium Chloride | 1.50 | Conditioning Agent |
| | Dehyquart Guar HP | Guar Hydroxypropyltrimoni um Chloride | 2.00 | Conditioning Agent |
| | Citric acid (50% solution) | Citric acid | 0.09 | Neutralizing agent |
| | Sodium Benzoate | Sodium Benzoate | 0.50 | Preservative |
| B | Dehyquart F 75 T | Distearoylethyl Hydroxyethylmonium Methosulfate, Cetearyl Alcohol | 1.00 | |
| | Lanette O | Cetearyl Alcohol | 3.00 | Consistency Agent |
| | Cetiol iSAN | | 2.00 | Emollient |
| C | PeptAlde 4.0 BC 10129 | Aqua, Hydrolyzed Rice Protein | 2.00 | Active Ingredient |
| | Perfume | Parfum | 0.40 | Fragrance |
| pH value 23°C | | | 5.0 | |

### 4.14 Mattifying cream

| **Ingredients** | **INCI** | **% by weight** | **Function** |
|---|---|---|---|
| Emulgade Sucro Plus | Sucrose Polystearate, Cetyl Palmitate | 3.00 | Emulsifier (O/W) |
| Cutina PES | Pentaerythrityl Distearate | 1.00 | Consistency agent |
| Myritol 318 | Caprylic/Capric Triglyceride | 2.00 | Emollient |
| Cetiol C 5C | Coco-Caprylate/Caprate | 2.00 | Emollient |
| Cetiol iSAN | Tridecane of example 1 | 3.00 | Emollient |
| Cetiol CC | Dicaprylyl Carbonate | 2.00 | Emollient |
| Cosmedia SP | Sodium Polyacrylate | 0.70 | Rheology modifier |
| Water, demin. | Aqua | 80.90 | |
| Glycerin | Glycerin | 2.00 | Humectant |
| Eumulgin SG | Sodium Stearoyl Glutamate | 0.50 | Emulsifier (O/W) |
| Preservative | | qs | Preservative |
| Bix'Activ BC 10050 | Bixa Orellana seed extract, Maltodextrin | 0.25 | Active Ingredient |
| Water, demin. | Aqua | 2.00 | |
| Perfume | Parfum | qs | Fragrance |
| Citric acid (10% solution) | Citric acid | 0.65 | pH Adjustment |

### 4.15 Well-aging lotion SPF 10

| **Ingredients** | **INCI** | **% by weight** | **Function** |
|---|---|---|---|
| Emulgade Sucro Plus | Sucrose Polystearate, Cetyl Palmitate | 2.50 | Emulsifier (O/W) |
| Eumulgin SG | Sodium Stearoyl Glutamate | 0.50 | Emulsifier (O/W) |
| Lanette O | Cetearyl Alcohol | 1.00 | Consistency agent |
| Cutina PES | Pentaerythrityl Distearate | 1.50 | Consistency agent |
| Cetiol OE | Dicaprylyl Ether | 3.00 | Emollient |
| Cetiol B | Dibutyl Adipate | 5.00 | Emollient |
| Cetiol Sensoft | Propylheptyl Caprylate | 5.00 | Emollient |
| Tinosorb S | Bis-Ethylhexyloxyphenol Methoxyphenyl Triazine | 1.50 | Broad spectrum UV filter |
| Water, demin. | Aqua | 65.40 | |
| Glycerin | Glycerin | 3.00 | Humectant |
| Rheocare XGN | Xanthan Gum | 0.30 | Stabilizer |
| Edeta BD | Disodium EDTA | 0.20 | Complexing agent |
| Tinosorb M | Methylene Bis-Benzotriazolyl Tetramethyl butyl phenol (nano), Aqua, Decyl Glucoside, Propylene Glycol, Xanthan Gum | 6.00 | Broad spectrum UV filter |
| Cetiol iSAN | Tridecane of example 1 | 2.00 | Emollient |
| Chione M SVA | Synthetic Fluorphlogopite, Lauroyl Lysine | 1.00 | Skin feel modifier |
| Water, demin. | Aqua | 2.00 | |
| Ciste'M BC 10023 | Cistus Monspeliensis Extract, Maltodextrin | 0.10 | Active Ingredient |
| Preservative | | qs | Preservative |

### 4.16 Aftershave - Lotion

| **Ingredients** | **INCI** | **% by weight** | **Function** |
|---|---|---|---|
| Water demin. | Aqua | 10.0 | |
| Cetiol iSAN | Tridecane of example 1 | 10.0 | Emollient |
| Pino SILVESTRE Classico After Shave fragrance | | 10.0 | Fragrance |
| Ethanol | | 70.0 | |

### 4.17 Caring liquid lip matte

| **Ingredients** | **INCI** | **% by weight** | **Function** |
|---|---|---|---|
| Dehymuls PGPH | Polyglyceryl-2 Dipolyhydroxystearate | 5.00 | Emulsifier W/O |
| Lameform TGI | Polyglyceryl-3 Diisostearate | 2.50 | Emulsifier W/O |
| Dehymuls SMS | Sorbitan Stearate | 3,00 | Emulsifier W/O |
| Cosmedia Gel CC | Dicaprylyl Carbonate, Stearalkonium Hectorite, Propylene Carbonate | 13,00 | Rheology modifier |
| Cetiol SB 45 | Butyrospermum Parkii Butter | 1.00 | Emollient |
| Cosmedia DC | Hydrogenated Dimer Dilinoleyl/Dimethylcarbonat e Copolymer | 5.00 | Film forming agent |
| Belsil PDM 1000 | Trimethylsiloxyphenyl Dimethicone | 5,00 | Emollient |
| Dow Corning MQ-1640 Flake Resin | Trimethylsiloxy Silicate, Polypropyl Silsesquioxane | 5.00 | Film forming agent |
| Cetiol Sensoft | Propylheptyl Caprylate | 3.00 | Emollient |
| Cetiol iSAN | Tridecane of example 1 | 3.00 | Emollient |
| Xiameter PMX-0245 Cyclopentasiloxa ne | Cyclopentasiloxane | 5.00 | Emollient |
| Irwinol LS 9890 | Octyldodecanol, Irvingia Gabonensis, Hydrogenated CocoGlycerides | 3.00 | Active Ingredient |
| Water, demin. | Aqua | 23.70 | |
| Glycerin | Glycerin | 7.00 | Humectant |
| Sodium Chloride | Sodium Chloride | 0,50 | Stabilizer |
| Euxyl PE 9010 | Phenoxyethanol, Ethylhexylglycerin | 1.10 | Preservative |
| Timica Terra White MN 4501 | Mica, Titanium Dioxide | 5.00 | Effect pigment |
| Timica Terra Red MN 4506 | Mica, Iron Oxides, Titanium Dioxide | 4.67 | Effect pigment |
| Timica Terra Black MN 44998 | Mica, Iron Oxides, Titanium Dioxide | 1.00 | Effect pigment |
| Timica Terra Yellow MN 4502 | Mica, Iron Oxides, Titanium Dioxide | 3.33 | Effect pigment |
| Covi Ox T 70 C | Tocopherol | 0.10 | Antioxidant |
| Perfume | Parfum | 0.10 | Fragrance |

### 4.18 Plump and moisturize lipstick

| **Ingredients** | **INCI** | **% by weight** | **Function** |
|---|---|---|---|
| Performalene 400 Polyethylene | Polyethylene | 6.80 | Structurant |
| Ozokerite Wax White SP 1020P | Ozokerite | 4.35 | Structurant |
| Microwax 190 | Microcrystalline wax | 2.00 | Structurant |
| Myritol 331 | Cocoglycerides | 15.65 | Emollient |
| Eutanol G | Octyldodecanol | 9.40 | Emollient |
| Myritol 312 | Caprylic/Capric Triglyceride | 5.05 | Emollient |
| Cetiol iSAN | Tridecane of example 1 | 2.50 | Emollient |
| Indopol H-100 | Polybutene | 9.00 | Film forming agent |
| KSG-16 | Dimethicone, Dimethicone/ Vinyl Dimethicone Crosspolymer | 9.00 | Skin feel modifier |
| Dow Corning 9041 Silicone Elastomer Blend | Dimethicone, Dimethicone Cross polymer | 9.00 | Skin feel modifier |
| Gransil PSQ | Polymethylsilsesquioxane | 2.20 | Skin feel modifier |
| HDK 2000 | Silica Silylate | 2.20 | Bulking agent |
| Dermol DISM | Diisostearyl Malate | 7.47 | Emollient |
| SunPURO black Iron oxide C33-7001 | CI 77499 | 0.20 | Colorant |
| Unicert Red K7061-J | CI 45380 | 0.09 | Colorant |
| Cloisonné Vibrant Raspberry F90H | Mica, Titanium Dioxide, Iron Oxides, Silica | 2.20 | Effect pigment |
| Reflecks^{™} Dimensions Glittering White G130S | Calcium Sodium Borosilicate (and) Titanium Dioxide | 0.75 | Effect Pigment |
| Reflecks^{™} Pinpoints of Pearl G130L | Calcium Sodium Borosilicate, Titanium Dioxide | 1.44 | Effect Pigment |
| Cosmedia DC | Hydrogenated Dimer Dilinoleyl/Dimethylcarbonate Copolymer | 1.65 | Film forming agent |
| Tinogard TL | Benzotriazolyl Dodecyl p-Cresol | 0.05 | Light stabilizer |
| Preservative | | 1.00 | Preservative |
| Ultra Filing spheres C00487 | Ethylhexyl Palmitate, Trihydroxystearin, Sodium Hyaluronate, Glucomannan | 3.00 | Active ingredient |
| AMC^{™} Advanced Moisture Complex NP | Glycerin, Aqua, Sodium PCA, Urea, Trehalose, Hexylene Glycol, Polyquaternium-51, Triacetin, Caprylyl Glycol, Sodium Hyaluronate | 5.00 | Active ingredient |

### 4.19 Anti-fatigue hybrid Concealer

| **Ingredients** | **INCI** | **% by weight** | **Function** |
|---|---|---|---|
| Dehymuls PGPH | Polyglyceryl-2 Dipolyhydroxystearate | 5.00 | Emulsifier W/O |
| Lameform TGI | Polyglyceryl-3 Diisostearate | 2.00 | Emulsifier W/O |
| Cutina PES | Pentaerythrityl Distearate | 1.50 | Consistency agent |
| Cosmedia Gel CC | Dicaprylyl Carbonate, Stearalkonium | 6.00 | Rheology modifier |
| | Hectorite, Propylene Carbonate | | |
| Cetiol Sensoft | Propylheptyl Caprylate | 6.00 | Emollient |
| Cetiol iSAN | Tridecane of example 1 | 2.00 | Emollient |
| KSG-016F | Dimethicone, Dimethicone/ Vinyl Dimethicone Crosspolymer | 2.00 | Skin feel modifier |
| Cetiol A | Hexyl laurate | 4.00 | Emollient |
| Cetiol C 5C | Coco-Caprylate/Caprate | 4.00 | Emollient |
| DK-PGT Paste Ti | Polyglyceryl-2 Triisostearate, Titanium Dioxide, Aluminum Hydroxide | 3.50 | Colorant |
| Water, demin. | Aqua | 55.70 | |
| Magnesium sulfate | Magnesium sulfate | 1.00 | Stabilizer |
| Butylene Glycol | Butylene Glycol | 2.00 | Humectant |
| Preservative | | qs | Preservative |
| Chione M SVA | Synthetic Fluorphlogopite, Lauroyl Lysine | 2.00 | Skin feel modifier |
| Timica Terra Yellow MN4502 | Mica, Iron Oxides, Titanium Dioxide | 1.60 | Effect pigment |
| Timica Terra Red MN4506 | Mica, Iron Oxides, Titanium Dioxide | 0.50 | Effect pigment |
| Timica Terra Black MN4498 | Mica, Iron Oxides, Titanium Dioxide | 0.20 | Effect pigment |
| Inolexir BC10079 | Glycerin, Aqua, Inonotus Obliquus (Mushroom) Extract | 1.00 | Active ingredient |

## Claims

1. A hydrocarbon mixture comprising
at least 95% by weight of saturated linear C-13 hydrocarbon based on the sum of the hydrocarbons in the hydrocarbon mixture,
**characterized in that** the amount of C11/C12 is 0.05 to 1.5 % by weight based on the sum of the hydrocarbons in the hydrocarbon mixture and
the amount of C14 to C17 is 0.2 to 3.5 % by weight based on the sum of the hydrocarbons in the hydrocarbon mixture.

2. The hydrocarbon mixture according to claim 1, **characterized in that** the hydrocarbon mixture comprises at least 95 to 99.5 % by weight of saturated linear C-13 hydrocarbon based on the sum of the hydrocarbons
**characterized in that** the amount of C11/C12 is 0.1 to 1.0 % by weight based on the sum of the hydrocarbons in the hydrocarbon mixture and
the amount of C14 to C17 is 0.3 to 3.0 % by weight based on the sum of the hydrocarbons in the hydrocarbon mixture.

3. The hydrocarbon mixture according to claim 1, **characterized in that** the hydrocarbon mixture comprises at least 97 % by weight of saturated linear C-13 hydrocarbon based on the sum of the hydrocarbons
**characterized in that** the amount of C11/C12 is 0.1 to 0.5 % by weight based on the sum of the hydrocarbons in the hydrocarbon mixture and
the amount of C14 to C17 is 0.4 to 2.5 % by weight based on the sum of the hydrocarbons in the hydrocarbon mixture.

4. The hydrocarbon mixture according to either of the preceding claims, **characterized in that** the **sum of the aromatic hydrocarbons** is less than or equal to 1 % by weight, especially less than or equal to 0.1 % by weight based on the sum of the hydrocarbons.

5. The hydrocarbon mixture according to any of the preceding claims, **characterized in that** the **sum of the unsaturated hydrocarbons** is less than or equal to 1 % by weight, especially less than or equal to 0.1 % by weight, especially less than or equal to 0.03 % by weight based on the sum of the hydrocarbons.

6. The hydrocarbon mixture according to any of the preceding claims, **characterized in that** the amount of tetradecanol is less than or equal to 1 % by weight based on the weight of the hydrocarbon mixture.

7. The hydrocarbon mixture according to any of the preceding claims, **characterized in that** the sum of the hydrocarbons having a **carbon chain length greater than or equal to 17** is less than or equal to 0.5 % by weight, based on the sum of the hydrocarbons.

8. The hydrocarbon mixture according to any of the preceding claims, **characterized in that** the sum of the hydrocarbons having **a carbon chain length less than or equal to 10 is less than** or equal to 0.5% by weight, based on the sum of the hydrocarbons.

9. The use of a hydrocarbon mixture according to any of claims 1 to 8 as emollient, toner, cleanser, conditioner, solvent, dispersant, oil bodies and/or dispersants in cosmetic and/or pharmaceutical formulations.

10. The use according to claim 9 in decorative or sun care cosmetic compositions.

11. A cosmetic formulation comprising 0.1 to 80% by weight of a hydrocarbon mixture according to any of claims 1 to 8.

12. The cosmetic formulation according to claim 11, comprising at least one perfume, perfume oil or fragrance.

13. The cosmetic formulation according to claim 11 which is free of perfumes, perfume oils or fragrances.

14. The cosmetic formulation according to any of claims 11 to 13, comprising at least one pigment and/or dye.

15. The cosmetic formulation according to any of claims 11 to 14, comprising at least one UV light protection filter.
